# EUROPEAN PATENT SPECIFICATION

(11) **EP 3 237 636 B1**
(45) Date of publication and mention of the grant of the patent: **21.10.2020**
(21) Application number: 15820430.5
(22) Date of filing: 22.12.2015
(51) Int. Cl.: C12Q 1/6818, C12Q 1/6851

(54) **DUAL QUENCHING ASSAY FOR MULTIPLEX DETECTION OF TARGET NUCLEIC ACIDS**
DUAL-QUENCHING VERFAHREN ZUR MULTIPLEX DETEKTION VON ZIELNUKLEINSÄUREN
DOSAGE PAR DOUBLE EXTINCTION POUR LA DÉTECTION MULTIPLEXE D'ACIDES NUCLÉIQUES CIBLES

(30) Priority: 22.12.2014 DK 201470813
(43) Date of publication of application: 01.11.2017
(73) Proprietor: Anapa Biotech A/S, 2970 Hørsholm (DK)
(72) Inventor: SCHNEIDER, Uffe Vest, 2500 Valby (DK); ECHWALD, Søren Morgenthaler, 3050 Humlebæk (DK); MIKKELSEN, Nikolaj Dam, 2700 Brønshøj (DK)
(74) Representative: Høiberg P/S
(86) International application number: PCT/DK2015/050412
(87) International publication number: WO 2016/101959

(56) References cited:
- EP-A1- 2 787 077
- WO-A1-2013/115442
- WO-A1-2013/133561
- US-A1- 2007 059 690

### SEQUENCE LISTING

<110> Anapa Biotech A/S
<120> Multiplex PCR
<130> P3649PC00
<160> 21
<170> PatentIn version 3.5
<210> 1
   <211> 20
   <212> DNA
   <213> Artificial sequence
<220>
   <223> Primer
<220>
   <221> misc_feature
   <222> (1)..(1)
   <223> TINA (twisted intercalating nucleic acid)
<400> 1
   gtccatcagg catcagaagg 20
<210> 2
   <211> 22
   <212> DNA
   <213> Artificial sequence
<220>
   <223> Primer
<220>
   <221> misc_feature
   <222> (1)..(1)
   <223> TINA (twisted intercalating nucleic acid)
<400> 2
   ggtagacttc tatctcatcc ac 22
<210> 3
   <211> 57
   <212> DNA
   <213> Artificial sequence
<220>
   <223> Quenching probe
<220>
   <221> misc_feature
   <222> (50)..(50)
   <223> BHQ1= black hole quencher 1
<220>
   <221> misc_feature
   <222> (54).. (54)
   <223> BHQ2= black hole quencher 2
<220>
   <221> misc_feature
   <222> (57).. (57)
   <223> BHQ3= black hole quencher 3
<400> 3
   gatactagag tttcatagtt cgtagtcaag atgatagatt ggaagtgcgt cagtcag 57
<210> 4
   <211> 48
   <212> DNA
   <213> Artificial sequence
<220>
   <223> Tagging probe
<220>
   <221> misc_feature
   <222> (1)..(1)
   <223> BHQ1= black hole quencher 1
<220>
   <221> misc_feature
   <222> (12)..(12)
   <223> FAM
<220>
   <221> misc_feature
   <222> (48)..(48)
   <223> phosphate
<400> 4
   aatgttccgc ctcgaaattc tggagtatat cgactgacgc acttccaa 48
<210> 5
   <211> 48
   <212> DNA
   <213> Artificial sequence
<220>
   <223> Tagging probe
<220>
   <221> misc_feature
   <222> (6)..(6)
   <223> BHQ1= black hole quencher 1
<220>
   <221> misc_feature
   <222> (18)..(18)
   <223> FAM
<220>
   <221> misc_feature
   <222> (48)..(48)
   <223> phosphate
<400> 5
   aatgttccgc ctcgaaattc tggagtatat cgactgacgc acttccaa 48
<210> 6
   <211> 48
   <212> DNA
   <213> Artificial sequence
<220>
   <223> Tagging probe
<220>
   <221> misc_feature
   <222> (1)..(1)
   <223> BHQ1= black hole quencher 1
<220>
   <221> misc_feature
   <222> (18)..(18)
   <223> FAM
<220>
   <221> misc_feature
   <222> (48)..(48)
   <223> phosphate
<400> 6
   aatgttccgc ctcgaaattc tggagtatat cgactgacgc acttccaa 48
<210> 7
   <211> 48
   <212> DNA
   <213> Artificial sequence
<220>
   <223> Tagging probe
<220>
   <221> misc_feature
   <222> (1)..(1)
   <223> BHQ1= black hole quencher 1
<220>
   <221> misc_feature
   <222> (21)..(21)
   <223> FAM
<220>
   <221> misc_feature
   <222> (48)..(48)
   <223> phosphate
<400> 7
   aatgttccgc ctcgaaattc tggagtatat cgactgacgc acttccaa 48
<210> 8
   <211> 48
   <212> DNA
   <213> Artificial sequence
<220>
   <223> Tagging probe
<220>
   <221> misc_feature
   <222> (1)..(1)
   <223> BHQ1= black hole quencher 1
<220>
   <221> misc_feature
   <222> (26)..(26)
   <223> FAM
<220>
   <221> misc_feature
   <222> (48)..(48)
   <223> phosphate
<400> 8
   aatgttccgc ctcgaaattc tggagtatat cgactgacgc acttccaa 48
<210> 9
   <211> 25
   <212> DNA
   <213> Artificial sequence
<220>
   <223> TaqMan probe
<220>
   <221> misc_feature
   <222> (1)..(1)
   <223> FAM
<220>
   <221> misc_feature
   <222> (25)..(25)
   <223> BHQ1= black hole quencher 1
<400> 9
   aatgttccgc ctcgaaattc tggag 25
<210> 10
   <211> 48
   <212> DNA
   <213> Artificial sequence
<220>
   <223> Tagging probe
<220>
   <221> misc_feature
   <222> (1)..(1)
   <223> BHQ1= black hole quencher 1
<220>
   <221> misc_feature
   <222> (12)..(12)
   <223> FAM
<220>
   <221> misc_feature
   <222> (48)..(48)
   <223> phosphate
<400> 10
   aatgttccgc ctcgaaattc tggagtatac tgactgacgc acttccaa 48
<210> 11
   <211> 57
   <212> DNA
   <213> Artificial sequence
<220>
   <223> Tagging probe
<220>
   <221> misc_feature
   <222> (1)..(1)
   <223> BHQ1= black hole quencher 1
<220>
   <221> misc_feature
   <222> (12)..(12)
   <223> FAM
<220>
   <221> misc_feature
   <222> (57).. (57)
   <223> phosphate
<400> 11
   aatgttccgc ctcgaaattc tggagtatac tgactgacgc acttccaatc tatcatc 57
<210> 12
   <211> 66
   <212> DNA
   <213> Artificial sequence
<220>
   <223> Tagging probe
<220>
   <221> misc_feature
   <222> (1)..(1)
   <223> BHQ1= black hole quencher 1
<220>
   <221> misc_feature
   <222> (12)..(12)
   <223> FAM
<220>
   <221> misc_feature
   <222> (66)..(66)
   <223> phosphate
<400> 12
   aatgttccgc ctcgaaattc tggagtatac tgactgacgc acttccaatc tatcatcttg 60
actacg 66
<210> 13
   <211> 59
   <212> DNA
   <213> Artificial sequence
<220>
   <223> Tagging sequence
<220>
   <221> misc_feature
   <222> (1)..(1)
   <223> BHQ1= black hole quencher 1
<220>
   <221> misc_feature
   <222> (12)..(12)
   <223> FAM
<220>
   <221> misc_feature
   <222> (59)..(59)
   <223> phosphate
<400> 13
   aatgttccgc ctcgaaattc tggagatatc gaacgcgaaa aaaaaaaaaa acgcgttcg 59
<210> 14
   <211> 49
   <212> DNA
   <213> Artificial sequence
<220>
   <223> Tagging probe
<220>
   <221> misc_feature
   <222> (1)..(1)
   <223> BHQ1= black hole quencher 1
<220>
   <221> misc_feature
   <222> (12)..(12)
   <223> FAM
<220>
   <221> misc_feature
   <222> (47)..(47)
   <223> BHQ1= black hole quencher 1
<220>
   <221> misc_feature
   <222> (49).. (49)
   <223> phosphate
<400> 14
   aatgttccgc ctcgaaattc tggagatatc gaacgcgaaa acgcgttcg 49
<210> 15
   <211> 360
   <212> DNA
   <213> Artificial sequence
<220>
   <223> RMD sequence
<220>
   <221> misc_feature
   <222> (1)..(360)
   <223> RMD sequence
<400> 15
   gaggaccgtg tcgcgctcac atggaacaat ctccggaaaa ccctcctggt ccatcaggca 60
   tcagaaggcc ttttcgataa tgataccggc gctctgctct ccctgggcag ggaaatgttc 120
   cgcctcgaaa ttctggagga cattgcccgg gataaagtca gaactctcca ttttgtggat 180
   gagatagaag tctacctggc cttccagacc atgctcgcag agaaacttca gctctctact 240
   gccgtgaagg aaatgcgttt ctatggcgtg tcgggagtga cagcaaatga cctccgcact 300
   gccgaagcca tggtcagaag ccgtgaagag aatgaattta cggactggtt ctccctctgg 360
<210> 16
   <211> 57
   <212> DNA
   <213> Artificial sequence
<220>
   <223> Quencher probe
<220>
   <221> misc_feature
   <222> (50)..(50)
   <223> BHQ1= black hole quencher 1
<220>
   <221> misc_feature
   <222> (54).. (54)
   <223> BHQ2= black hole quencher 2
<220>
   <221> misc_feature
   <222> (57).. (57)
   <223> BHQ3= black hole quencher 3
<400> 16
   gatactagag tttcatagtt cgtagtcaag atgatagatt ggaagtgcgt cagtcag 57
<210> 17
   <211> 46
   <212> DNA
   <213> Artificial sequence
<220>
   <223> Probe
<220>
   <221> misc_feature
   <222> (1)..(1)
   <223> BHQ1= black hole quencher 1
<220>
   <221> misc_feature
   <222> (12)..(12)
   <223> FAM
<220>
   <221> misc_feature
   <222> (46)..(46)
   <223> phosphate
<400> 17
   aatgttccgc ctcgaaattc tggagtatac ggccgattag atatag 46
<210> 18
   <211> 51
   <212> DNA
   <213> Artificial sequence
<220>
   <223> Probe
<220>
   <221> misc_feature
   <222> (1)..(1)
   <223> BHQ1= black hole quencher 1
<220>
   <221> misc_feature
   <222> (12)..(12)
   <223> FAM
<220>
   <221> misc_feature
   <222> (51)..(51)
   <223> phosphate
<400> 18
   aatgttccgc ctcgaaattc tggagtatac ggccgattag atatagaatg g 51
<210> 19
   <211> 58
   <212> DNA
   <213> Artificial sequence
<220>
   <223> Probe
<220>
   <221> misc_feature
   <222> (1)..(1)
   <223> BHQ1= black hole quencher 1
<220>
   <221> misc_feature
   <222> (12)..(12)
   <223> FAM
<220>
   <221> misc_feature
   <222> (58)..(58)
   <223> phosphate
<400> 19
   aatgttccgc ctcgaaattc tggagtatac ggccgattag atatagaatg gatatcgc 58
<210> 20
   <211> 74
   <212> DNA
   <213> Artificial sequence
<220>
   <223> Probe
<220>
   <221> misc_feature
   <222> (1)..(1)
   <223> BHQ1= black hole quencher 1
<220>
   <221> misc_feature
   <222> (12)..(12)
   <223> FAM
<220>
   <221> misc_feature
   <222> (74)..(74)
   <223> phosphate
<400> 20
   aatgttccgc ctcgaaattc tggagtatac ggccgattag atatagaatg gatatcgcta       60
   tagatcttat tcgg                                                         74
<210> 21
   <211> 91
   <212> DNA
   <213> Artificial sequence
<220>
   <223> Probe
<220>
   <221> misc_feature
   <222> (1)..(1)
   <223> BHQ1= black hole quencher 1
<220>
   <221> misc_feature
   <222> (12)..(12)
   <223> FAM
<220>
   <221> misc_feature
   <222> (91).. (91)
   <223> phosphate
<400> 21
   aatgttccgc ctcgaaattc tggagtatac ggccgattag atatagaatg gatatcgcta       60
   tagatcttat tcggttaaga tagttgtagg c                                      91

## Description

### Field

The present disclosure relates to a qPCR method for indirect detection of multiple target DNA sequences based on melting temperature determination. The present invention further relates to a kit of parts.

### Background

Detection of specific sequences in a DNA sample by PCR has become a standard process. The technique is used for a range of different purposes from gene deletion analysis to pathogen identification and template quantitation. Typically fluorophores of different colors are used to detect different targets. However, due to the limited number of fluorophores which can easily be distinguished from one another, this gives a limitation in the number of specific targets that can be detected.

Furthermore, the step of DNA hybridization during the PCR reaction is affected by ionic strength, base composition, length of fragment to which the nucleic acid has been reduced, the degree of mismatching, and the presence of denaturing agents. DNA hybridization-based technologies are very useful tools in nucleic acid sequence determination and in clinical diagnosis, genetic research, and forensic laboratory analysis. A disadvantage, however, is that most of the conventional methods depending on hybridization are likely to produce false positive results due to nonspecific hybridization between probes and non-target nucleic acid sequences. Therefore, there remain problems to be solved for improving their reliability.

Seegene, Seoul, Korea has developed a technology which can also accommodate multiplexing by melting curve analysis. As described in WO2013115442A1, Seegene's TOCE technology is based on a probe which releases a primer fragment upon hydrolysis. This fragment is then required to act as a primer on a second, artificial target, where a doublestranded target is generated, having a specific melting profile which can be linked to that particular probe. While this system can also accommodate high multiplexing by melting, it is inherently more complex than the present disclosure, by requiring the released fragment to initiate and complete a second extension on an artificial target. The fragment generated in the present disclosure will directly provide a labelled, melting fragment.
WO2013133561 describes detection of a nucleotide variant on a target nucleic acid sequence by a PTOCE (PTO Cleavage and Extension) assay.

Pathofinder BV, Maastricht, Holland has developed a technology, which can also accommodate multiplexing by melting curve analysis. As described in United States Patent Application 20100297630 A1, this system is based on providing 2 target specific probes which, when hybridized adjacently on the target, can be conjoined by a ligase, which produces a melt-able fragment with a melting profile which is specific for the specific target probes. However, as indicated in United States Patent Application 20100297630 A1, this system is not a truly homogeneous assay but requires the sample tubes to be opened after the first PCR reaction to add e.g. a ligase solution. This extra step is not required in the method of the present disclosure and is highly undesired due to the risk of contamination by PCR product and also involves an additional handling step.

Roche Diagnostics has developed a method and assay for sepsis detection which also accommodates multiplexing by melting curve analysis. This system relies on FRET probes, where 2 probes having interacting labels are designed to bind adjacently to a single target, where the fluorophore of the first probe interacts with the second probe to generate a signal. The 2 probes can be designed such that the probes can generate a specific melting curve when subjected to a temperature gradient. A disadvantage by this system is that the melting profile must be designed within the specific target sequence, where the method of the present disclosure provides melting tags which, once optimized, can be attached to any target specific probes. In addition, since FRET probes require 2 labels, such a system can accommodate a lower level of multiplexing on a PCR system with e.g. 5 fluorophore detection channels compared to the present disclosure which carries one flourophore per probe.

Other methods for multiplex DNA target detection are dependent on solid surface conjugation e.g. by chip based probes. Even though these chip based methods may be able to distinguish numerous target nucleic acid sequences, the chip-assembly process is cumbersome and often involve complicated, expensive and delicate equipment.

Therefore, there remains a need for convenient, reliable, and reproducible detection of multiple target nucleic acid sequences. Furthermore, a novel target detection method not limited by the number of fluorescent labels is needed. In addition there is a need in to reduce the complexity and number of steps in multiplex nucleic acid sequence analysis and thus facilitate more cost-effective and simple clinical diagnostic methods, genetic research protocols, and forensic laboratory analyses.

### Summary

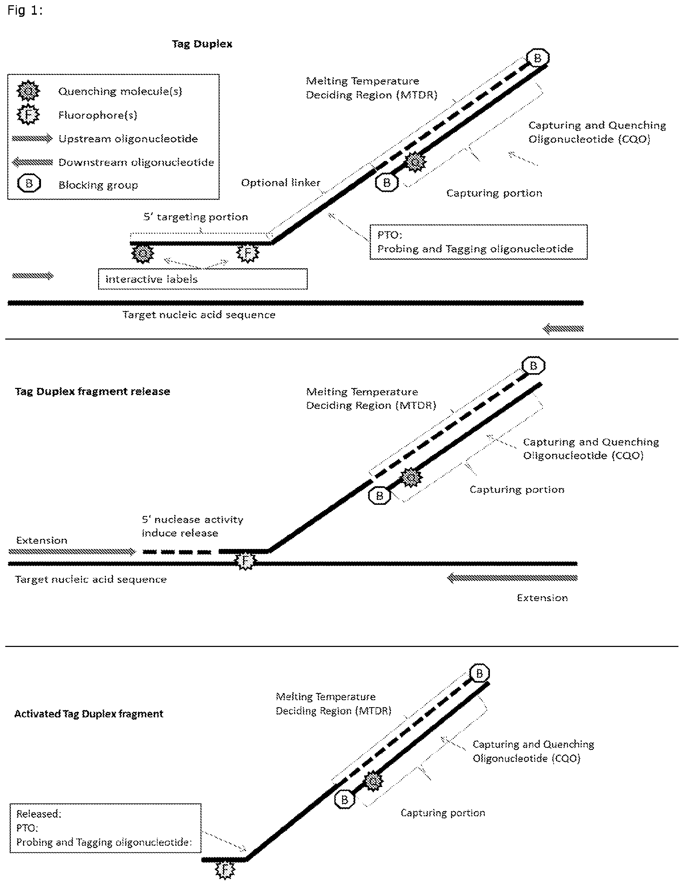

The present disclosure provides a simple, convenient, reliable, and reproducible method of detecting multiple target DNA sequences. The present disclosure uses melting curve determinations for the detection of several target sequences per fluorophore. By this method a multitude of targets can be detected with a single fluorophore and/or a large number of targets can be detected by both employing different melting temperatures and different fluorophores. The present inventors have developed a dual quenched assay in combination with melting curve determinations for multiplex detection of target DNA sequences using different probing and tagging oligonucleotides (PTOs) each comprising a different melting temperature dependent region (MTDR) together with a single capturing and quenching oligonucleotide (CQO). Using several CQOs and PTOs of the present disclosure further increase the number of target sequence which can be detected in a single assay. A concept of the present disclosure, termed the MeltPlex system, is illustrated in figure 1.

The present disclosure prevents false positive detection of target nucleic acid sequences by a combination of: 1) sequence specific hybridization to a target nucleic acid sequence and 2) sequence specific enzymatic release of an activated Tag Duplex fragment required for target signal detection (figure 1). This indirect measurement of target nucleic acid sequences through the presence of an activated Tag Duplex fragment ensures excellent accuracy and reduces if not completely overcomes the issues with false positive results.

One advantage of the MeltPlex system is that one CQO may detect multiple target sequences identified by several unique PTOs. For each target nucleic acid sequence to be detected, a PTO with a MTDR sequence (melting temperature dependent region) which is unique within PTO's with same fluorophore, is designed. Consequently the number of target nucleic acid sequences which can be detected using only a single fluorescent label is increased. Different PTOs with similar fluorophores that are compatible with the same CQO may be referred to as a PTO group since they contain similar and/or identical fluorescent labels. Each PTO group may be detected by a single CQO, which forms an activated Tag Duplex, wherein the activated Tag Duplex fragments are distinguished based on differences in melting temperature as a consequence of the unique MTDR on each PTO in the group. Several PTO groups may also be detected by a single CQO, optionally by including more than on quencher in the CQO to allow quenching of a broad range of fluorophores by the same CQO.. Hence the present disclosure is able to increase the number of target nucleic acid sequences which can be detected in an assay without requiring different types of fluorescent labels for each detected nucleic acid target nucleic acid sequence. The present disclosure may apply several fluorescent labels for different PTO groups, which further increases the number of target nucleic acid sequences which can be detected in an assay using standard laboratory equipment.

In addition the CQOs of the present disclosure are independent of the target sequences. Thus CQOs, which have proven to yield reliable results in some assays can be re-used in other assays. Such re-use of probes may save significant resources when designing new assays.

Contamination is a major issue in PCR based technologies. One option to limit the risk of contamination is to use a technology which does not require re-opening of the reaction vial after the assay has started. The present disclosure does not require re-opening of the reaction vials after assay start and is consequently less prone to contaminations.

A major aspect of the present disclosure relates to a method for detecting a target nucleic acid sequence, said method comprises the steps of:
(a) hybridizing the target nucleic acid sequence with a PTO (Probing and Tagging Oligonucleotide); the PTO comprising (i) a targeting portion comprising a nucleotide sequence substantially complementary to the target nucleic acid sequence, and (ii) a Melting Temperature Deciding Region (MTDR), comprising a nucleotide sequence non-complementary to the target nucleic acid sequence, and (iii) at least one set of interactive labels comprising at least one fluorophore and at least one quencher; wherein the targeting portion of the PTO can hybridize with the target nucleic acid sequence and the MTDR of the PTO is not hybridized with the target nucleic acid sequence;
(b) hybridizing said PTO with a CQO (Capturing and Quenching Oligonucleotide); wherein the CQO comprises (i) a capturing portion comprising a nucleotide sequence which is reverse complementary to the MTDR of the PTO and (ii) at least one quenching molecule; wherein the MTDR of the PTO is configured to hybridize with the capturing portion of the CQO to form a Tag Duplex;
(c) contacting the Tag Duplex with an enzyme having nuclease activity; wherein the enzyme having nuclease activity induces cleavage of the Tag Duplex when the Tag Duplex is hybridized with the target nucleic acid sequence thereby releasing an activated Tag Duplex fragment comprising a PTO fragment comprising the MTDR hybridized to the capturing portion of the CQO and the at least one fluorophore, wherein the PTO fragment is hybridized with the capturing portion of the CQO;
(d) melting and/or hybridizing said activated Tag Duplex fragment to obtain a signal from the at least one fluorophore, and;
(e) detecting the activated Tag Duplex fragment by measuring the signal from the at least one fluorophore; wherein the signal is indicative of the presence of the target nucleic acid sequence.

Steps a) and b) of the method may occur in any order, i.e. the Tag duplex may be formed prior to the binding of the PTO / Tag duplex to the target nucleic acid.

In another embodiment of the present disclosure, steps (c) and (b) are switched so the method comprises the steps of (a) hybridizing the PTO and target, (c) contacting the PTO and target with enzyme having nuclease activity thus releasing the activated PTO and (b) hybridizing the activated PTO with a CQO thus forming an activated Tag Duplex and then steps (d) and (e) follow as disclosed above.

The steps of the method may be repeated.

The presence of activated PTO or activated Tag Duplex is registered.

The temperature at which the Tag Duplex melts (step d) is registered.

The assay of the present disclosure has a multitude of applications. A non-exhaustive list of applications includes:
- Human and/or veterinary diagnostics
- Food and/or feed quality and safety
- Environmental surveillance
- Scientific research

The assay of the present disclosure may be sold as a kit of parts. Thus an aspect of the present disclosure relates to a kit of parts for detection of a target nucleic acid sequence as described herein, the kit comprising:
i. optionally at least one PTO described herein, and
ii. at least one CQO described herein, and
iii. instructions on how to detect a target nucleic acid sequence.

An embodiment of the present disclosure is thus the detection of one or more target nucleic acid sequences with a single CQO.

### Description of Drawings

**Fig 1****:** A non-limiting illustration of one embodiment of the present disclosure.

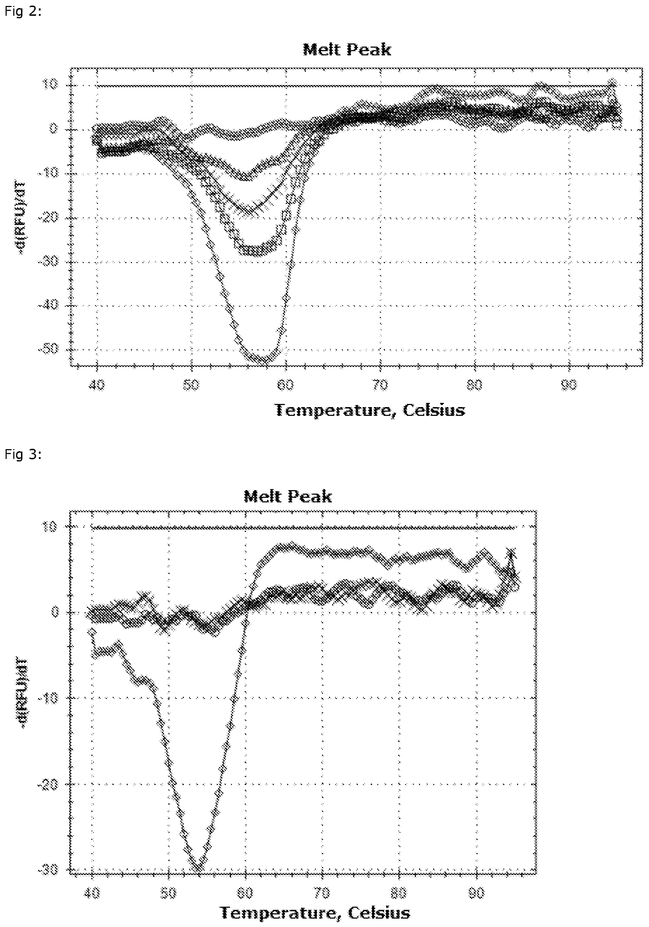

**Fig 2****:** Melting curve analysis of NTC (no template control) PCR reaction products: DEC486P does not generate a melting curve in the negative control as desired, whereas all other designs show increasing levels of melting curve signal in NTC reactions. (Results were performed in triplicates - singleplex shown). ○: DEC486P, Δ: DEC487P, ×: DEC488P, □: DEC489P, ◊: DEC490P.
**Fig 3****:** Melting curve analysis of PCR reaction products with and without template for tagging probe DEC486P. NTC (X) does not generate a melting curve in the negative control, whereas there is a clearly distinguishable signal from the reaction with template (◊). Also, no melting curve is seen in reaction without quenching probe (○). (Results were performed in triplicates - singleplex shown).

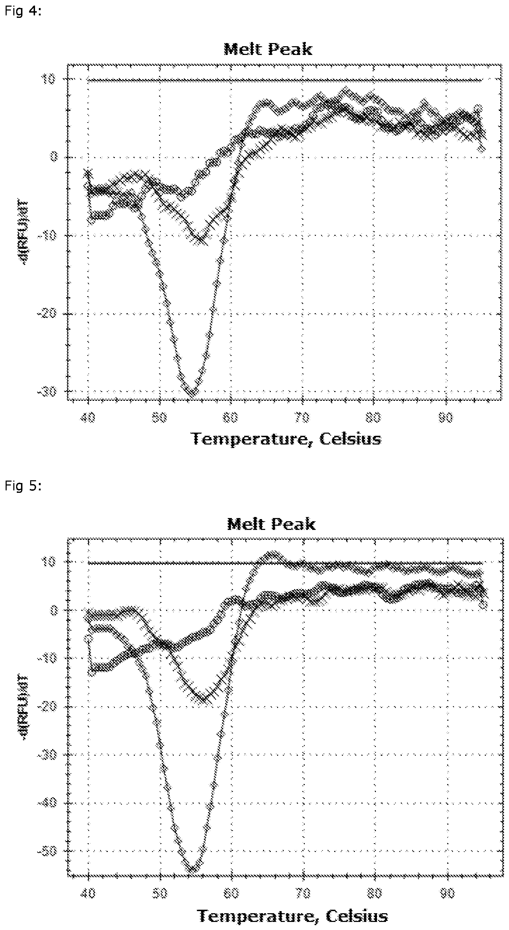

**Fig 4****:** Melting curve analysis of PCR reaction products with and without template for tagging probe DEC487P. NTC (X) generates a small melting curve in the negative control, whereas there is a clearly distinguishable signal from the reaction with template (◊). No melting curve is seen in reaction without quenching probe (O). (Results were performed in triplicates - singleplex shown).
**Fig 5****:** Melting curve analysis of PCR reaction products with and without template for tagging probe DEC488P. NTC (X) generates a modest melting curve in the negative control, whereas there is a clearly distinguishable signal from the reaction with template (◊). No melting curve is seen in reaction without quenching probe (O). (Results were performed in triplicates - singleplex shown).

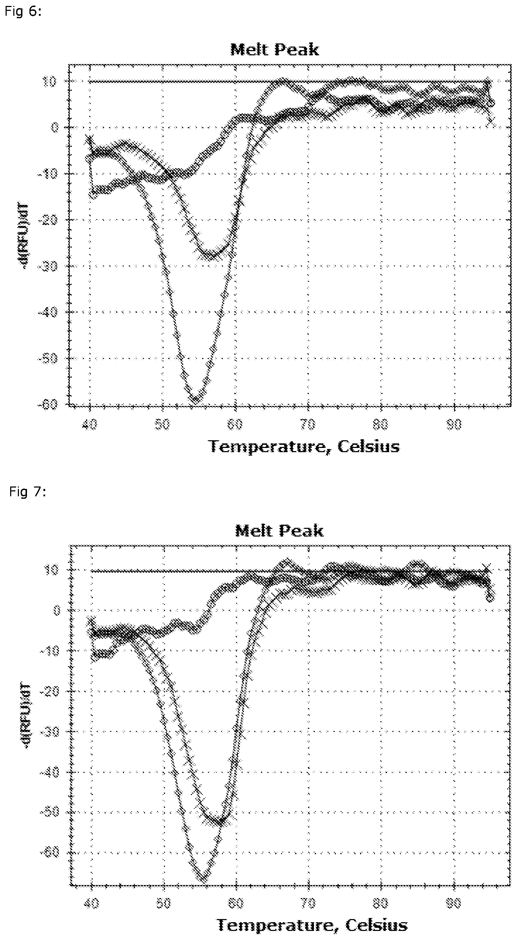

**Fig 6****:** Melting curve analysis of PCR reaction products with and without template for tagging probe DEC489P. NTC (X) generates a clear melting curve in the negative control, whereas there is a distinguishable signal from the reaction with template (◊). No melting curve is seen in reaction without quenching probe (O). (Results were performed in triplicates - singleplex shown).
**Fig 7****:** Melting curve analysis of PCR reaction products with and without template for tagging probe DEC490P. NTC (X) generates a significant melting curve in the negative control, which is not clearly distinguishable from the reaction with template (◊). No melting curve is seen in reaction without quenching probe (O). (Results were performed in triplicates - singleplex shown).

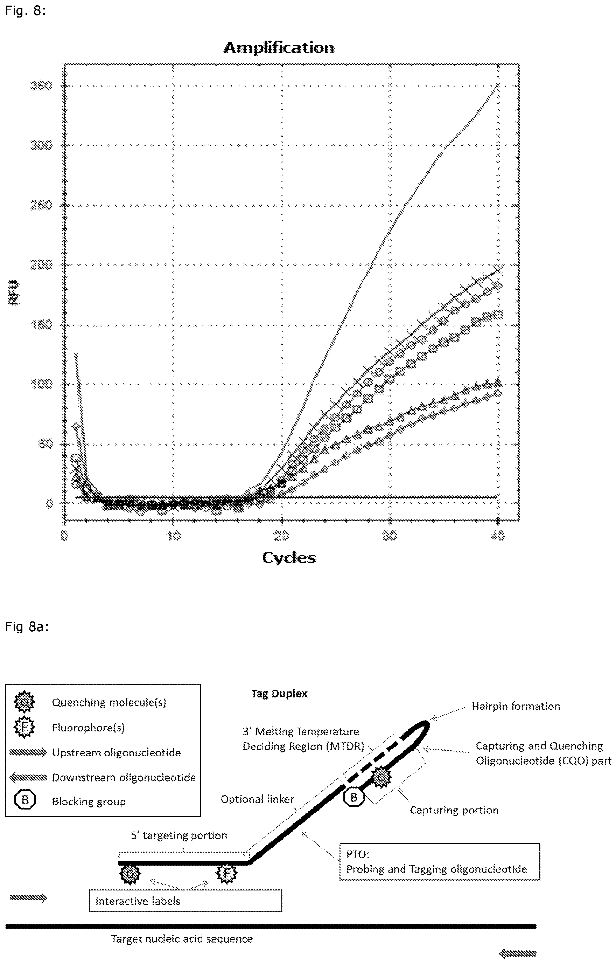

**Fig. 8****:** Q-PCR amplification curves for tagging probes DEC486P - DEC490P on positive samples. ○: DEC486P, Δ: DEC487P, ×: DEC488P, □: DEC489P, ◊: DEC490P, full line: DEC464. Although signal intensity is lower for tagging probes compared to the TaqMan-type probe, all probes clearly generate positive signal readouts with CT values of between 16 and 20.
**Fig. 8a****:** A non-limiting illustration of one embodiment of the present disclosure.

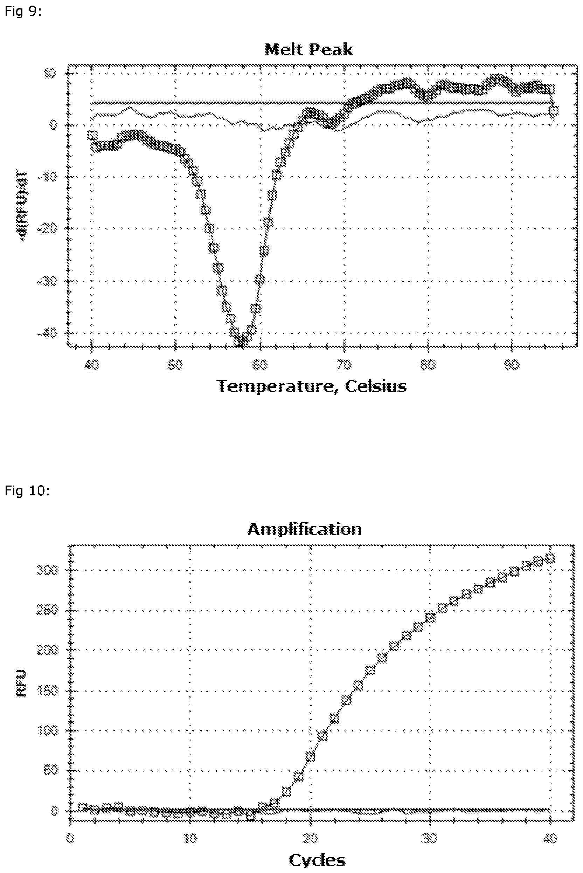

**Fig 9****:** Melting curve analysis of target positive and NTC (no template control) PCR reaction products: DEC500P generates a melting curve in the positive sample but not in the negative control as desired (results were performed in triplicates - singleplex shown). □ : DEC500P, -: DEC500P NTC (non template control)
**Fig 10****:** Amplification curve of target positive and NTC (no template control) PCR reaction products: DEC500P generates an amplification curve in the positive sample but not in the negative control as desired (results were performed in triplicates - singleplex shown). □ : DEC500P, -: DEC500P NTC (non template control)

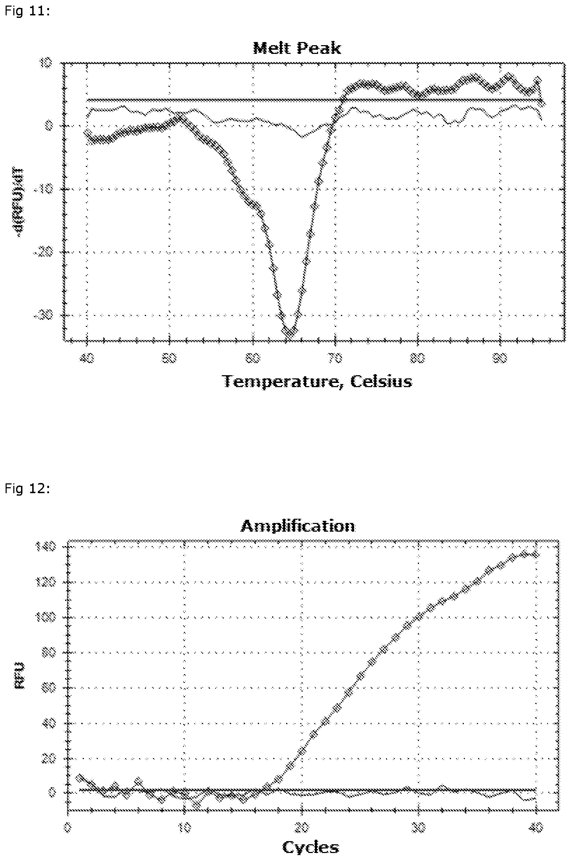

**Fig 11****:** Melting curve analysis of target positive and NTC (no template control) PCR reaction products: DEC502P generates a melting curve in the positive sample but not in the negative control as desired (results were performed in triplicates - singleplex shown). □: DEC500P, ◊: DEC502P, - :DEC502P NTC (non template control)
**Fig 12****:** Amplification curve of target positive and NTC (no template control) PCR reaction products: DEC502P generates an amplification curve in the positive sample but not in the negative control as desired (results were performed in triplicates - singleplex shown). □: DEC500P, ◊: DEC502P, - :DEC502P NTC (non template control)

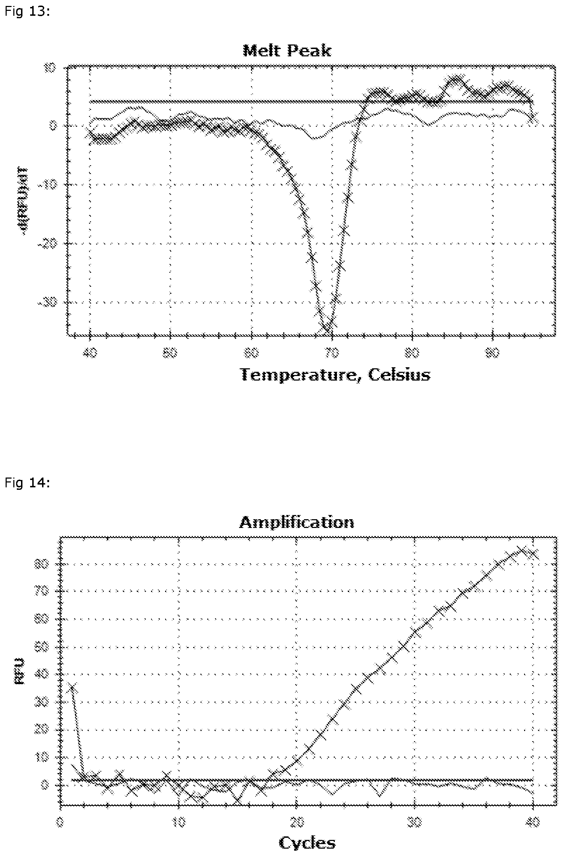

**Fig 13****:** Melting curve analysis of target positive and NTC (no template control) PCR reaction products: DEC503P generates a melting curve in the positive sample but not in the negative control as desired (results were performed in triplicates - singleplex shown). ×: DEC503P, - : DEC503P NTC (non template control)
**Fig 14****:** Amplification curve of target positive and NTC (no template control) PCR reaction products: DEC503P generates an amplification curve in the positive sample but not in the negative control as desired (results were performed in triplicates - singleplex shown). ×: DEC503P, - : DEC503P NTC (non template control)

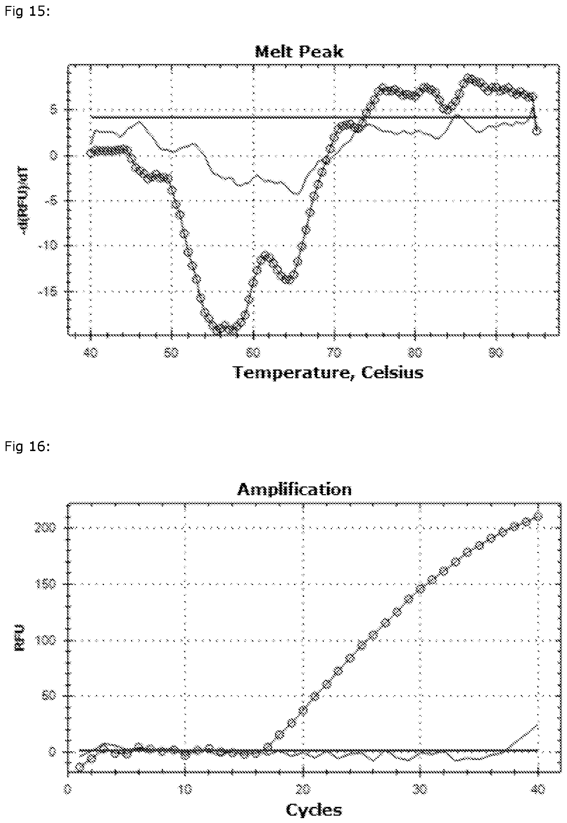

**Fig 15****:** Melting curve analysis of target positive and NTC (no template control) PCR reaction using DEC500P and DEC502P generates 2 individually distinguishable melting curve in the positive sample but not in the negative control as desired (results were performed in triplicates - singleplex shown). ○: DEC500P + DEC 502P, - : DEC500P + DEC 502P NTC (non template control).
**Fig 16****:** Amplification curve of target positive and NTC (no template control) PCR reaction products using DEC500P and DEC502P generates a amplification curve in the positive sample but not in the negative control as desired (results were performed in triplicates - singleplex shown). ○: DEC500P + DEC 502P, - : DEC500P + DEC 502P NTC (non template control).

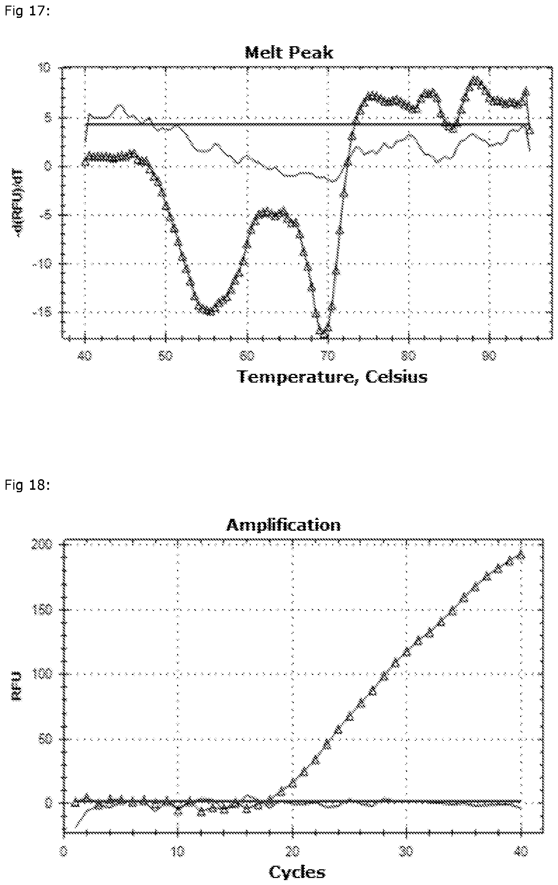

**Fig 17****:** Melting curve analysis of target positive and NTC (no template control) PCR reaction using DEC500P and DEC503P generates 2 individually distinguishable melting curve in the positive sample but not in the negative control as desired (results were performed in triplicates - singleplex shown). Δ: DEC500P + DEC 503P, - : DEC500P + DEC 503P NTC (non template control)
**Fig 18****:** Amplification curve of target positive and NTC (no template control) PCR reaction products using DEC500P and DEC503P generates a amplification curve in the positive sample but not in the negative control as desired (results were performed in triplicates - singleplex shown). Δ: DEC500P + DEC 503P, - : DEC500P + DEC 503P NTC (non template control)

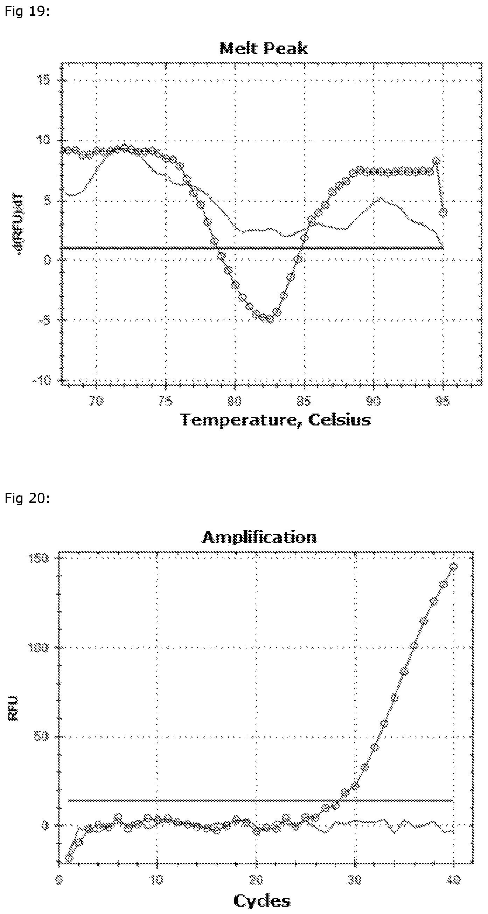

**Fig 19****:** Melting curve analysis of target positive and NTC (no template control) PCR reaction products: RMD7P generates a melting curve in the positive sample but not in the negative control as desired (results were performed in triplicates - singleplex shown). ○ : RMD7P, -: RMD7P NTC (non template control).
**Fig 20****:** Amplification curve of target positive and NTC (no template control) PCR reaction products: RMD7P generates an amplification curve in the positive sample but not in the negative control as desired (results were performed in triplicates - singleplex shown). ○ : RMD7P, -: RMD7P NTC (non template control).

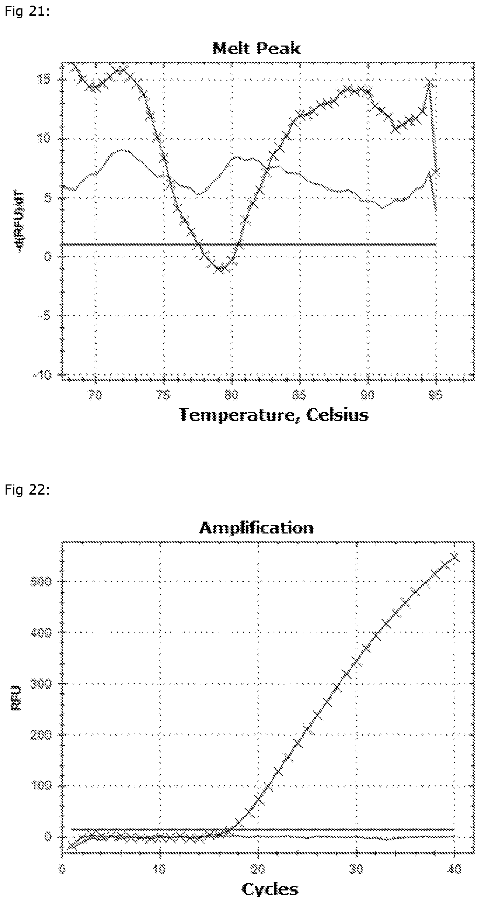

**Fig 21****:** Melting curve analysis of target positive and NTC (no template control) PCR reaction products: RMD8P generates a melting curve in the positive sample but not in the negative control as desired (results were performed in triplicates - singleplex shown). X: RMD8P, -: RMD8P NTC (non template control).
**Fig 22****:** Amplification curve of target positive and NTC (no template control) PCR reaction products: RMD8P generates an amplification curve in the positive sample but not in the negative control as desired (results were performed in triplicates - singleplex shown). X: RMD8P, -: RMD8P NTC (non template control).

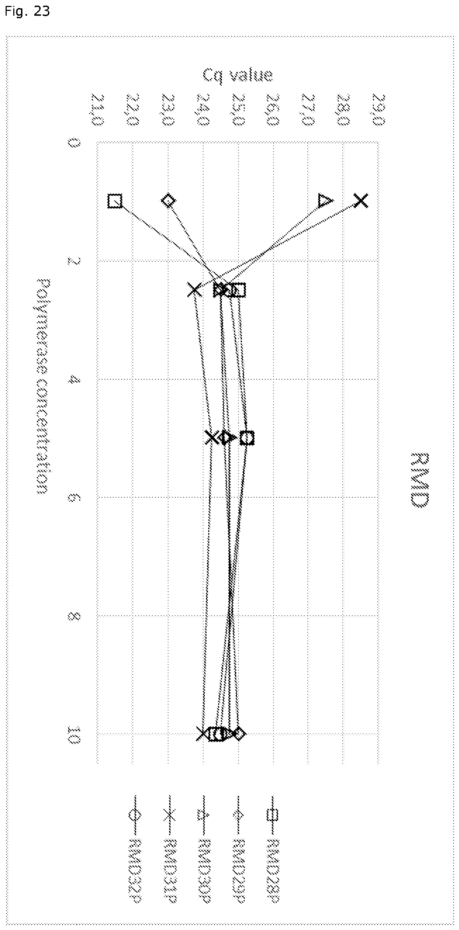

**Fig. 23****.** Averaged Cq values of the RMD assay, plotted as a function of polymerase concentration. X-axis: arbitrary units.

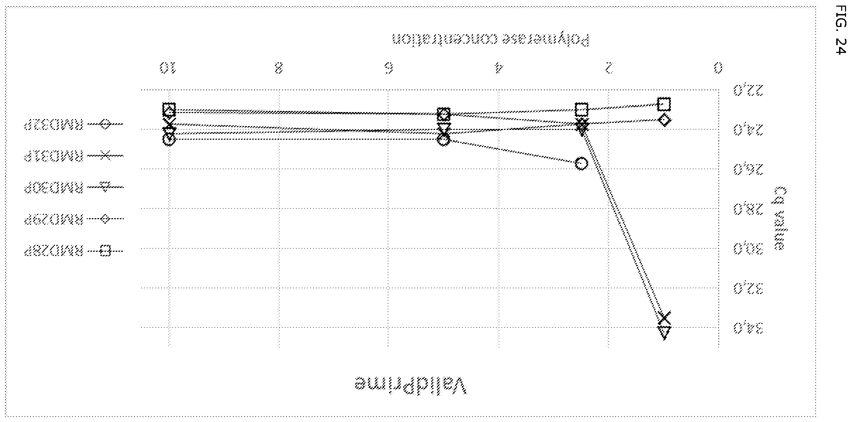

**Fig. 24****.** Averaged Cq values of the mValidPrime assay, plotted as a function of polymerase concentration. X-axis: arbitrary unit, where 1 is the concentration recommended by the manufacturer.

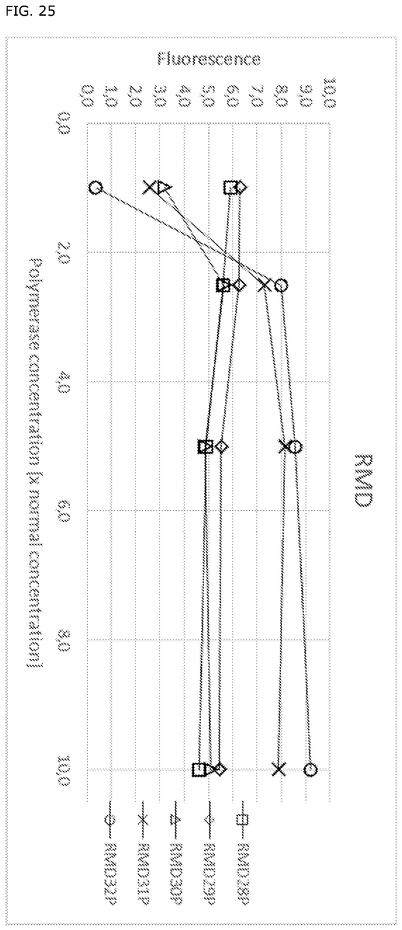

**Fig. 25****.** Amplitude of the RMD amplification curves in step 2 (95°C) as a function of polymerase concentration. X-axis: arbitrary unit, where 1 is the concentration recommended by the manufacturer.

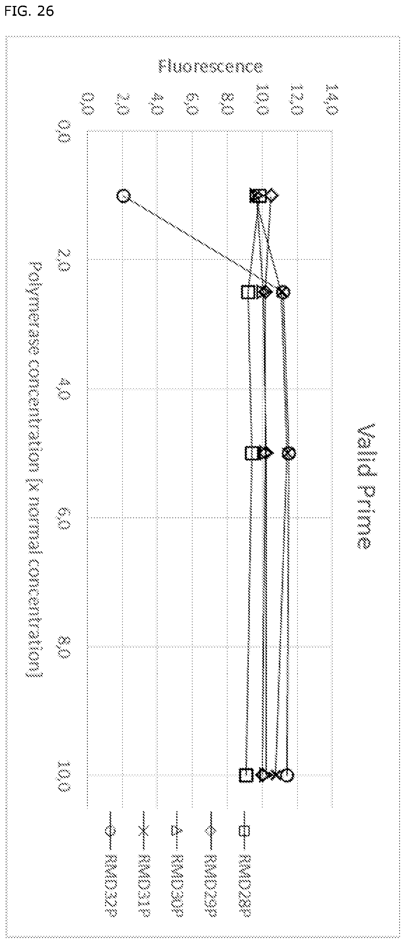

**Fig. 26****.** Amplitude of the mValidPrime amplification curves in step 2 (95°C) as a function of polymerase concentration. X-axis: arbitrary unit, where 1 is the concentration recommended by the manufacturer.

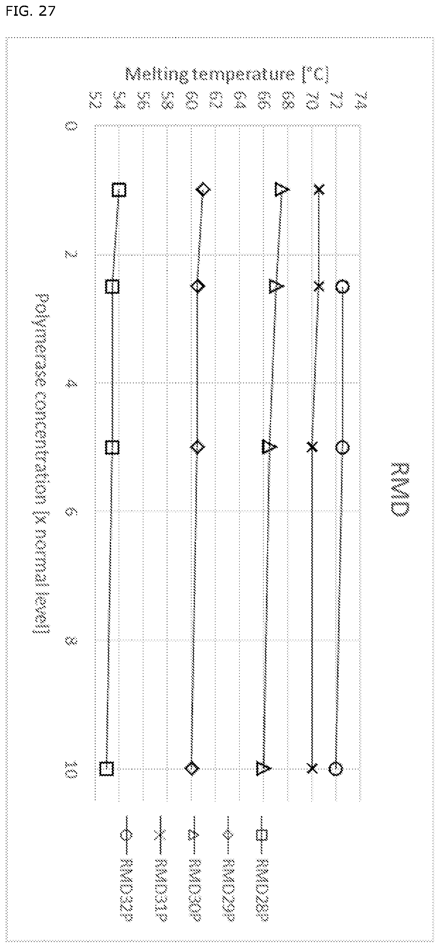

**Fig. 27****.** Melting temperatures of the RMD probe/quencher duplexes as a function of polymerase concentration. X-axis: arbitrary unit, where 1 is the concentration recommended by the manufacturer.

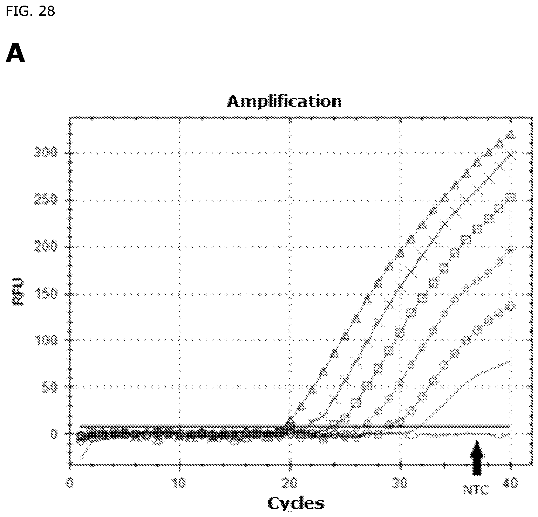

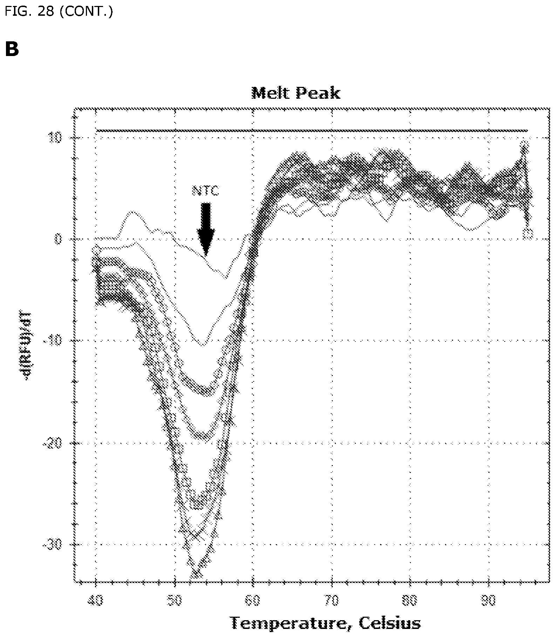

**Fig. 28****:** (A) Amplification curve of target positive and NTC (no template control) PCR reaction products: DEC486P generates amplification curves with Cq values in the positive samples corresponding to the target concentration but not in the negative control as desired (results were performed in duplicates - singleplex shown). Δ: 5x dilution, ×: 25x dilution, □ : 125x dilution, ◊: 625x dilution, O: 3125x dilution, - :15625x dilution, NTC: (non template control). (B) Melting curve analysis of target positive and NTC (no template control) PCR reaction products: DEC486P generates melting curves amplitudes in the positive samples corresponding to the target concentration but not in the negative control as desired (results were performed in duplicates - singleplex shown). Δ: 5x dilution, ×: 25x dilution, □ : 125x dilution, ◊: 625x dilution, O: 3125x dilution, - :15625x dilution, NTC: (non template control).

### Detailed description

The invention is defined by the appended claims. Any embodiment not falling under the scope of the appended claims does not form part of the invention.

The major challenges of multiplex PCR is easy detection of multiple target DNA sequences using a simple, convenient, and reliable method. The present inventors have developed a dual quenched assay in combination with melting curve determination for detection of several target DNA sequences per label, i.e. per fluorophore. A non-limiting concept of the present disclosure is illustrated in figure 1.

### Definitions

The term "double quenched assay" as used herein refers to the use of at least two quenchers for at least one fluorophore. In an embodiment of the present disclosure one quencher is situated on the CQO and another quencher is situated on the PTO. In an embodiment of the present disclosure the fluorophore is situated on the PTO.

The term "interactive labels" or "set of interactive labels" as used herein refers to at least one fluorophore and at least one quencher which can interact when they are located adjacently. When the interactive labels are located adjacently the quencher can quench the fluorophore signal. The interaction may be mediated by fluorescence resonance energy transfer (FRET).

The term "located adjacently" as used herein refers to the physical distance between two objects. If a fluorophore and a quencher are located adjacently, the quencher is able to partly or fully quench the fluorophore signal. FRET quenching may typically occur over distances up to about 100 Å. Located adjacently as used herein refers to distances below and/or around 100 Å.

The term "probing and tagging oligonucleotide" or "PTO" as used herein refers to an oligonucleotide comprising at least one set of interactive labels. A PTO of the present disclosure is configured to hybridize to a target nucleic acid sequence. A PTO comprises a targeting portion, a "Melting Temperature Deciding Region" or "MTDR" (see definition below), and optionally a linker between the targeting portion and the MTDR.

The term "PTO group" as used herein refers to a number of PTOs with the same set of interactive labels, wherein each PTO in the group has a unique targeting sequence and MTDR region. Each PTO in a group may be configured to detect different target nucleic acid sequences and the unique MTDR facilitates distinction of each PTO in the group by means of melting temperature as described herein.

The term "Capturing and quenching oligonucleotide" or "CQO" as used herein refers to an oligonucleotide comprising at least one quencher and a capturing portion. The capturing portion of the CQO is configured to hybridize to a PTO of the present disclosure.

The term "Tag duplex" as used herein refers to a PTO and a CQO which are hybridized. The PTO may furthermore be hybridized to a target nucleic acid sequence.

The term "Tag Duplex fragment" or "activated Tag Duplex fragment" or activated Tag Duplex as used herein refers to a PTO fragment and a CQO which are hybridized, wherein the quencher of the PTO is not present. The quencher of the PTO has been released as a consequence of the enzyme having nuclease activity which induces cleavage of the Tag Duplex and release of the PTO quencher. "Activated PTO" refers to the PTO where the quencher has been removed. The presence of activated PTO may be measured using qPCR and/or real-time PCR. Dependent on the sequence of the steps of the methods disclosed herein the presence of either activated PTO or activated Tag Duplex is measured. In preferred embodiments of the present disclosure only activated Tag Duplex can be detected by real time PCR. In the most preferred embodiments of the present disclosure only the activated Tag Duplex can be detected by real time PCR due to complete quenching of the PTO fluorophore by the CQO quencher. In other embodiments of the present disclosure the assay is calibrated after any signal detected by an activated PTO.

The term "fluorescent label" or "fluorophore" as used herein refers to a fluorescent chemical compound that can re-emit light upon light excitation. The fluorophore absorbs light energy of a specific wavelength and re-emits light at a longer wavelength. The absorbed wavelengths, energy transfer efficiency, and time before emission depend on both the fluorophore structure and its chemical environment, as the molecule in its excited state interacts with surrounding molecules. Wavelengths of maximum absorption (≈ excitation) and emission (for example, Absorption/Emission = 485 nm/517 nm) are the typical terms used to refer to a given fluorophore, but the whole spectrum may be important to consider.

The term "quench" or "quenching" as used herein refers to any process which decreases the fluorescence intensity of a given substance such as a fluorophore. Quenching may be mediated by fluorescence resonance energy transfer (FRET). FRET is based on classical dipole-dipole interactions between the transition dipoles of the donor (e.g. fluorophore) and acceptor (e.g. quencher) and is dependent on the donor-acceptor distance. FRET can typically occur over distances up to 100 Å. FRET also depends on the donor-acceptor spectral overlap and the relative orientation of the donor and acceptor transition dipole moments. Quenching of a fluorophore can also occur as a result of the formation of a non-fluorescent complex between a fluorophore and another fluorophore or non-fluorescent molecule. This mechanism is known as 'contact quenching, "static quenching,' or 'ground-state complex formation

The term "quencher" as used herein refers to a chemical compound which is able to quench a given substance such as a fluorophore.

In multiplex PCR more than one target nucleic acid sequence may be detected.

The term "Melting Temperature Deciding Region" or "MTDR" as used herein refers to a polynucleotide region located in the 5' end of the PTO. The nature and/or the number of polynucleotides in the MTDR are decisive for the melting temperature of e.g. the activated Tag Duplex comprising a PTO fragment and a CQO. Likewise the MTDR is decisive for the hybridization temperature of e.g. the activated Tag Duplex comprising a PTO fragment and a CQO.

The term "melting temperature" or "Tₘ" as used herein refers to the temperature at which one half of a DNA duplex will dissociate to become single stranded and thus indicates the duplex stability. The main factors affecting Tₘ are salt concentration, DNA concentration, pH and the presence of denaturants (such as formamide or DMSO). Other effects such as sequence, length, and hybridization conditions can be important as well. The GC content of the sequence and the salt concentration gives a fair indication of the primer Tₘ. The melting temperatures referred to in the present disclosure are calculated using the nearest neighbor thermodynamic theory as described by Kibbe et al. 2007. The corresponding Tₘ calculator is available at the URL: http://basic.northwestern.edu/biotools/OligoCalc.html. The Tₘ values given in the present disclosure have been calculated on the basis of 800 nm CQO ("Primer") and 50 nm (Na⁺). In melting temperature calculations of oligos comprising analogs of adenine, thymine, cytosine and/or guanine the analog is replaced by its corresponding nucleic acid. Fluorophore and quenchers on the oligos should not be considered when calculating the melting temperature. Determination of the melting temperature may be performed either by heating a DNA duplex or by cooling (hybridizing) two single stranded DNA strands which are substantially complementary.

The term "denaturation" as used herein is the dissociation by disrupting the hydrogen bonds between complementary bases of DNA to become single stranded. It may also refer to a cycling event of a PCR reaction and may e.g. comprise heating the reaction to 90-100 °C for 3-240 seconds.

The term "ready to use pellet" as used herein refers to a substantially water free composition comprising at least one PTO and/or at least one CQO of the present disclosure.

The term "background melting curve generation" as used herein refers to background signals during melting curve analysis. A background signal may occur if the signal of the at least one fluorophore on the PTO is not completely quenched by the at least one quencher of the PTO.

The term "TINA" as used herein, refers to a twisted intercalating nucleic acid and is a group of nucleic acid intercalating molecules as described in US patent 9,102,937.

The term "locked nucleic acid" (LNA) as used herein refers to a modified RNA nucleotide. The ribose moiety of an LNA nucleotide is modified with an extra bridge connecting the 2' oxygen and 4' carbon. The bridge "locks" the ribose in the 3'-endo (North) conformation, which is often found in the A-form duplexes. LNA nucleotides can be mixed with DNA or RNA residues in the oligonucleotide whenever desired and hybridize with DNA or RNA according to Watson-Crick base-pairing rules. Such oligomers are synthesized chemically and are commercially available. The locked ribose conformation enhances base stacking and backbone pre-organization. This significantly increases the hybridization properties of oligonucleotides by increasing the thermal stability of duplexes. LNAs and methods of synthesis thereof are known to the skilled person, and are described e.g. in European patents EP1015469 and EP1015469.

The term 'reverse complementary' as used herein designates a nucleic acid sequence which is capable of hybridizing to another nucleic acid sequence of which it is the reverse complement. For example, the reverse complement of a sequence 5'-N₁N₂N₃N₄... Nₓ-3' is 5'-Nₓ'...N₄'N₃'N₂'N₁'-3', where Nₓ', N₄', N₃', N₂', N₁' indicate the nucleotides complementary to Nₓ, N₄, N₃, N₂, N₁, respectively.

### Target detection

The present disclosure relates to a novel dual quenching assay which allows simultaneous detection of multiple target nucleic acids per fluorescent label. The presence of multiple target nucleic acid sequences in a sample results in the formation of multiple Tag Duplex fragments which can be distinguished based on differences in Tag Duplex fragment melting temperatures and/or hybridization temperature. The Tag Duplex comprises a PTO and a CQO which are hybridized. When each activated Tag Duplex fragment melts, a signal from a label of the PTO is obtained. A signal at a specific temperature is thus indicative of the presence of the target sequence that the PTO is specific for. When each activated Tag Duplex fragment hybridizes, a signal from a label of the PTO is quenched. A quenched signal at a specific temperature is thus indicative of the presence of the target sequence that the PTO is specific for. The general concept of the present disclosure is illustrated in figure 1. The present disclosure prevents false positive signals by a combination of 1) sequence specific hybridization to the target nucleic acid sequence by means of the PTO targeting portion and 2) sequence specific enzymatic release of an activated Tag Duplex fragment required for target signal detection, by means of the nuclease activity of a polymerase extending an oligonucleotide upstream of the target sequence. This indirect measurement of the target nucleic acid sequence through the presence of an activated Tag Duplex fragment ensures excellent accuracy and overcomes issues with false positives. A single CQO may hybridize to many PTOs and/or many groups of PTOs. Thus the present disclosure relates to the detection of one or more, such a multiple, nucleic acids with the aid of a single CQO.

### Melting temperature mediated identification of multiple target nucleic acid sequences

Using one CQO for detection of several PTO's with unique MTDRs results in a simpler assay setup which can detect several target nucleic acid sequences per fluorophore (e.g. per PTO group). The number of PTOs in a PTO group which can be distinguished using one fluorophore is dependent of the sensitivity of the analytical equipment used for detecting the signal of the fluorescent tag upon melting the activated Tag Duplex fragment. In a simple setup provided here by way of example: one CQO may be used to identify at least three PTOs of a PTO group; using two PTO groups with two different fluorophores may thus facilitate detection of at least 6 PTOs in a single reaction. Each PTO indicates the presence of a target nucleic acid sequence. The number of targets to be identified may be further increased by using three or more PTO groups with different fluorescent tags. A single CQO may of course be able to hybridize with more than two, such as three, four or more PTOs. A PTO group may comprise 2, 3, 4, 5, 6, 7 or more PTOs and a single CQO may be used to detect each of these PTOs. Simultaneously other PTO groups (PTOs with different flourophores) may be detected with the same CQO as well. In this manner a single CQO may be used to detect multiple targets. In an embodiment of the present disclosure a single CQO may thus detect 2, 4, 6, 8, 10, 15, 20, 30, 40, 50, or more PTOs and thus the assay can detect the corresponding number of target nucleic acids.

The setup of the dual quenching assay of the present disclosure yields a simple reliable assay for multiplex detection of target nucleic acids. The simple assay of the present disclosure has a multitude of applications. A non-exhaustive list of applications of the assay of the present disclosure may be:
- The use of the method of the present disclosure for human and/or veterinary diagnostics. E.g. clinical identification and/or quantification of a target nucleic acid from at least one microorganism, such as a pathogen, or oncogene or microsatellite in a bodily sample from a subject.
- The use of the method of the present disclosure for environmental surveillance. E.g. identification and/or quantification of a target nucleic acid from at least one (micro)organism in any sample, for example a water sample.
- The use of the method of the present disclosure for food and/or feed quality and safety determination e.g. identification and/or quantification of a target nucleic acid from at least one organism in any sample such as a feed and/or food product, such as a beverage sample.
- The use of the method of the present disclosure for scientific research.

In a main aspect the present disclosure relates to a method for detecting a target nucleic acid sequence, the method comprising the steps of:
Step (a) hybridizing a target nucleic acid sequence with a PTO (Probing and Tagging Oligonucleotide); the PTO comprising (i) a targeting portion comprising a nucleotide sequence substantially complementary to the target nucleic acid sequence, and (ii) a Melting Temperature Deciding Region (MTDR), comprising a nucleotide sequence non-complementary to the target nucleic acid sequence, and (iii) at least one set of interactive labels comprising at least one fluorophore and at least one quencher;
Step (b) hybridizing said PTO with a CQO (Capturing and Quenching Oligonucleotide); wherein the CQO comprises (i) a capturing portion comprising a nucleotide sequence which is reverse complementary to the MTDR of the PTO and (ii) at least one quenching molecule; wherein the MTDR of the PTO is configured to hybridize with the capturing portion of the CQO to form a Tag Duplex;
Step (c) contacting the Tag Duplex with an enzyme having nuclease activity; wherein the enzyme having nuclease activity induces cleavage of the Tag Duplex when the Tag Duplex is hybridized with the target nucleic acid sequence thereby releasing an activated Tag Duplex fragment comprising a PTO fragment comprising the MTDR hybridized to the capturing portion of the CQO and the at least one fluorophore;
Step (d) melting and/or hybridizing said activated Tag Duplex fragment to obtain a signal from the at least one fluorophore, and
Step (e) detecting the activated Tag Duplex fragment by measuring the signal from the at least one fluorophore; wherein the signal is indicative of the presence of the target nucleic acid sequence.

Steps (a), (b) and (c) of any of the herein embodiments of the present disclosure of the method may form part of a PCR reaction. A set of oligonucleotide primers is added that will amplify the target sequence. This amplification will due to the presence of a polymerase with exonuclease activity result in the release of the thus activated PTO or the activated Tag Duplex. The term activated relates to the absence of the quencher on the PTO. Any presence of activated PTO or activated Tag Duplex may be registered / detected. If the PCR reaction is a qPCR reaction the amount of activated PTO or activated Tag Duplex may be quantified. The oligonucleotide primer pair comprises a first a primer complementary to said target nucleic acid and which primes the synthesis of a first extension product that is complementary to said target nucleic acid, and a second primer complementary to said first extension product and which primes the synthesis of a second extension product. The PTO may hybridize to the target nucleotide sequence and inter alia to the amplification product.

In an embodiment of the present disclosure the presence of activated PTO and/or activated Tag Duplex is detected. In another embodiment of the present disclosure the amount of activated PTO and/or activated Tag Duplex is quantified. The detection and/or quantification of the presence of activated PTO and/or activated tag Duplex may be an optional step to be included once the activated PTO and/or activated Tag Duplex is formed.

Steps (d) and (e) form part of a melting assay in which the presence and/or absence targets is determined based on the Tm of the activated Tag Duplex. The melting assay may be run in a PCR machine.

Steps (a) and (b) may occur in any order. Thus in an embodiment of the present disclosure the method described herein comprises the steps of:
Step (b) hybridizing a PTO (Probing and Tagging Oligonucleotide) with a CQO (Capturing and Quenching Oligonucleotide); the PTO comprising (i) a targeting portion comprising a nucleotide sequence substantially complementary to the target nucleic acid sequence, and (ii) a Melting Temperature Deciding Region (MTDR), comprising a nucleotide sequence non-complementary to the target nucleic acid sequence, and (iii) at least one set of interactive labels comprising at least one fluorophore and at least one quencher; and wherein the CQO comprises (i) a capturing portion comprising a nucleotide sequence which is reverse complementary to the MTDR of the PTO and (ii) at least one quenching molecule; wherein the MTDR of the PTO is configured to hybridize with the capturing portion of the CQO to form a Tag Duplex;
Step (a) hybridizing a target nucleic acid sequence with a PTO of a Tag Duplex;
Step (c) contacting the Tag Duplex with an enzyme having nuclease activity; wherein the enzyme having nuclease activity induces cleavage of the Tag Duplex when the Tag Duplex is hybridized with the target nucleic acid sequence thereby releasing an activated Tag Duplex fragment comprising a PTO fragment comprising the MTDR hybridized to the capturing portion of the CQO and the at least one fluorophore;
Step (d) melting and/or hybridizing said activated Tag Duplex fragment to obtain a signal from the at least one fluorophore, and
Step (e) detecting the activated Tag Duplex fragment by measuring the signal from the at least one fluorophore; wherein the signal is indicative of the presence of the target nucleic acid sequence.

In yet an embodiment of the present disclosure steps (b) and (c) occur in reverse order. Thus in an embodiment of the present disclosure the method described herein comprises the steps of:
Step (a) hybridizing a target nucleic acid sequence with a PTO (Probing and Tagging Oligonucleotide); the PTO comprising (i) a targeting portion comprising a nucleotide sequence substantially complementary to the target nucleic acid sequence, and (ii) a Melting Temperature Deciding Region (MTDR), comprising a nucleotide sequence non-complementary to the target nucleic acid sequence, and (iii) at least one set of interactive labels comprising at least one fluorophore and at least one quencher;
Step (c) contacting the hybridized PTO with an enzyme having nuclease activity; wherein the enzyme having nuclease activity induces cleavage of the PTO when the PTO is hybridized with the target nucleic acid sequence thereby releasing an activated PTO fragment comprising the MTDR and the at least one fluorophore;
Step (b) hybridizing said activated PTO with a CQO (Capturing and Quenching Oligonucleotide); wherein the CQO comprises (i) a capturing portion comprising a nucleotide sequence which is reverse complementary to the MTDR of the PTO and (ii) at least one quenching molecule; wherein the MTDR of the PTO is configured to hybridize with the capturing portion of the CQO to form an activated Tag Duplex;
Step (d) melting and/or hybridizing said activated Tag Duplex fragment to obtain a signal from the at least one fluorophore, and
Step (e) detecting the activated Tag Duplex fragment by measuring the signal from the at least one fluorophore; wherein the signal is indicative of the presence of the target nucleic acid sequence.

In all the assays the steps may be repeated. Particularly steps (a) and (c) in the orders indicated above for various assays may be repeated one or more times. The number of repetitions may be as is customary for performing PCR reactions.

The temperature at which the Tag Duplex melts in step (d) of the method is registered. Thus in an embodiment of the disclosure step (d) comprises:
Step (d) melting and/or hybridizing said activated Tag Duplex fragment to obtain a signal from the at least one fluorophore, and registering the melting temperature of the activated Tag Duplex fragment.

In an embodiment of the present disclosure the method described above further comprises repeating the steps (a)-(b), (a)-(c), (a)-(d) and/or (a)-(e) with denaturation between repeating cycles. It follows that in an embodiment of the present disclosure starting with step (b), steps (b)-(a), (b)-(c), and/or (b) to (e) are repeated with denaturation between repeating cycles. In another embodiment of the present disclosure the steps are performed in one reaction vessel or some of the steps (a)-(e) or (b)-(e) are performed in one or more separate reaction vessels. In an embodiment of the present disclosure starting with steps (a), (c) and (b) the steps may be repeated by repeating steps (a)-(c), (a)-(b), (a)-(d) and/or (a)-(e) with denaturation between repeating cycles. In a further embodiment of the present disclosure of all the methods, the steps (a), (b) and (c) occur simultaneously or more or less simultaneously and/or steps (d) and (e) occur simultaneously or more or less simultaneously.

A non-limiting example illustrating the detection of two target sequences may be:
A mixture comprising:
1) a PTO#1 comprising a targeting portion#1, and a MTDR#1 configured to yield a melting temperature of 50°C, wherein the PTO#1 can hybridize to target nucleic acid sequence#1, and
2) a PTO#2 comprising a targeting portion#2, and a MTDR#2 configured to yield a melting temperature of 60°C, wherein the PTO#2 can hybridize to a target nucleic acid sequence#2, and
3) a CQO which is configured to hybridize to MDTR#1 and MDTR#2 as described herein above.

In presence of the target nucleic acid sequence#1 and sequence#2 the method of the present disclosure yields an activated Tag Duplex fragment#1 wherein the CQO is hybridized to the MTDR#1 of the PTO#1 and an activated Tag Duplex fragment#2 wherein the CQO is hybridized to the MTDR#2 of the PTO#2. When melting a mixture comprising the activated Tag Duplex fragment#1 and Tag Duplex fragment#2 over a range of temperatures a first signal is generated when the temperature reaches 50°C and a second signal is generated when the temperature reaches 60°C. In this simplified example the first signal (at 50 °C) is indicative of the presence of the target nucleic acid sequence#1 and the second signal is indicative of the presence of the of target nucleic acid sequence#2. Thus with one fluorophore it is possible to detect at least two different targets. A person of skill can easily see how a multitude of targets may be determined by the method of the present disclosure.

The present disclosure may further include at least one Tag Duplex or DNA duplex comprising at least one set of interactive labels comprising at least one fluorophore and at least one quencher which may be used as control sample for example for calibrating the Tₘ of the analytical equipment. The Tₘ of an oligonucleotide may vary depending on e.g. the salt concentration, DNA concentration, pH and the presence of denaturants (such as formamide or DMSO). Inclusion of a control sample may be desirable if the samples to be analyzed contain varying i.e. salt concentrations. In an embodiment of the present disclosure the method described herein further comprises analyzing a control sample and/or control Tag Duplex. In another embodiment of the present disclosure the method described herein further comprises analyzing a control sample and/or control Tag Duplex to calibrate the Tₘ output of the analytical equipment. It follows that in the absence of target sequence the present methods will detect the presence of PTO and tag Duplexes that are not activated by the removal of the quencher on the PTO. As is known to the person of skill: when running assays as those disclosed herein a negative control (no target present) and a positive control (presence of target) may be included. The controls may be used to calibrate the assay. An example of a commercially available control for the presence of genomic DNA is ValidPrime: http://www.tataa.com/wp-ontent/uploads/2012/10/TATAA-Manual ValidPrime Probe v01 1.pdf.

As is known to the person skilled in the art, the melting temperature of a duplex is usually not dependent on the nature of the sequence, but rather on the relative amounts of the individual nucleotides. The skilled person also knows to avoid particular sequences which might result in the generation of secondary or tertiary structures which might impede the reaction. As illustrated in the examples, the present methods work with MTDR having different sequences.

The MTDR comprises a nucleotide sequence non-complementary to the target nucleic acid sequence. The term 'non-complementary' in this context will be understood by the skilled person as referring to a sequence which is essentially non-complementary, i.e. essentially unable to hybridize to the target nucleic acid sequence under normal PCR conditions and/or stringent conditions.

Similarly, the term 'a nucleotide sequence substantially complementary to the target nucleic acid sequence' refers to a nucleotide sequence which is able to hybridize to the target nucleic acid sequence in such a manner that extension by a polymerase is efficient or even feasible. As will be obvious to the skilled person, there may be some mismatches, provided that they do not prevent hybridization of the nucleotide sequence to the target nucleic acid sequence to such an extent that extension by a polymerase is not possible.

### PTO

For each target nucleic acid sequence to be identified a PTO configured for hybridizing to each target nucleic acid sequence is obtained. Each PTO has a MTDR which is unique among PTO sharing same or similar fluorophore. The MTDR of the PTO is decisive for the activated Tag Duplex fragment melting temperature. Each Tag Duplex fragment comprises a PTO fragment with a unique MTDR and the at least one fluorophore, wherein the MTDR of the PTO fragment is hybridized with the capturing portion of the CQO. By unique MTDR is meant an MTDR which is unique in the melting temperature it confers to the Tag Duplex and/or Tag Duplex fragment. The unique MTDR is thus unique within a group of PTOs. A unique MTDR may thus be used in several PTOs each with different labels / fluorophores.

In an embodiment of the present disclosure the targeting portion is located in the 5' end of the PTO. In another embodiment of the present disclosure the MTDR is located in the 3' end of the PTO.

In another embodiment of the present disclosure the PTO comprises non-nucleic acid molecules and or nucleic acid analogs.

### Melting temperature deciding region (MTDR)

The length of the MTDR of the PTO which forms the activated Tag Duplex fragment alters the melting temperature of said Tag Duplex fragment. The use of short MTDR regions (e.g. shorter than 10 nucleic acids) will yield a low melting temperature. The inventors have used MTDR of various lengths such as around 16 to around 40 nucleic acids. However the MTDR region of the present disclosure may comprise 5-100 nucleic acids and/or nucleic acid analogues, such as 10 - 80, such as 15-70, preferably such as 13-60, more preferably such as 16-39 nucleic acids and/or nucleic acid analogues. Thus an embodiment of the present disclosure the MTDR comprises 5-50 nucleic acids and/or nucleic acid analogues, such as 10 - 40, such as 13-30 nucleic acids and/or nucleic acid analogues. When using long MTDR regions (e.g. more than 50 nucleic acids) care should be taken to avoid secondary structures forming within the MTDR itself. In a preferred embodiment of the present disclosure the MTDR region of the present disclosure comprises 13-25 nucleic acids and/or nucleic acid analogues such as locked nucleic acids (LNA). Specific examples of nucleic acid analogs also include, but are not limited to, the following bases in base pair combinations: iso-C/iso-G, iso-dC/iso-dG.

In an embodiment of the present disclosure, step (a) comprises hybridizing the target nucleic acid sequence with a PTO; the PTO comprising (i) a targeting portion comprising a hybridizing nucleotide sequence substantially complementary to the target nucleic acid sequence, and (ii) a MTDR, comprising a nucleotide sequence non-complementary to the target nucleic acid sequence, wherein the MDTR comprises 5-50 nucleic acids and/or nucleic acid analogues, such as 10 - 40, such as 13-30 nucleic acids and/or nucleic acid analogues, and (iii) at least one set of interactive labels comprising at least one fluorophore and at least one quencher; wherein the targeting portion of the PTO is configured to hybridize with the target nucleic acid sequence and MTDR of the PTO is not configured to hybridize with the target nucleic acid sequence.

One advantage of the present disclosure is that one CQO may detect multiple target sequences identified by unique PTOs. For each target nucleic acid sequence to be detected a PTO with an MTDR sequence is designed which is unique for PTOs having same or similar fluorescent labels, these different PTOs may be referred to as a PTO group if they contain similar and/or identical fluorescent labels. Each PTO group may be detected by a single CQO, which forms an activated Tag Duplex fragment, wherein the activated Tag Duplex fragments are distinguished based on differences in melting temperature as a consequence of the unique MTDR on each PTO in the group. Consequently the number of target nucleic acid sequences which can be detected using only a single fluorescent label is increased.

In another embodiment of the present disclosure, for each distinguishable fluorescent label, the method described herein can distinguish at least one, such as at least two, such as at least three, such as at least four, such as at least five, such as at least ten target nucleic acid sequences from each other based on the difference in melting temperature of their respective Tag Duplex fragments, wherein the melting temperature of each Tag Duplex fragment is determined by the length and composition of the MTDR. In another embodiment of the present disclosure the length and/or the composition of the MTDR as described herein determines the melting temperature of the activated Tag Duplex fragment described herein above. The melting temperature of the activated Tag Duplex fragment may be any temperature; however a temperature above room temperature is preferable. PCR reactions are conducted in aqueous buffers which typically have a boiling point near 100°C. Thus in an embodiment of the present disclosure the melting temperature of the activated Tag Duplex fragment described herein is between 30°C to 100°C, such as between 35°C to 90°C, such as between 40°C to 75°C such as between 45°C to 75°C. In a preferred embodiment of the present disclosure the melting temperature of the activated Tag Duplex fragment is between 35°C to 90°C, such as between 50°C to 85°C. In a further embodiment of the present disclosure the MTDRs of the PTO forming Tag Duplex fragment is configured to yield a melting temperature of the activated Tag Duplex fragment between 30°C to 100°C, such as between 35°C to 80°C, such as between 40°C to 75°C such as between 45°C to 75°C. In a preferred embodiment of the present disclosure the MTDRs of the PTO forming Tag Duplex fragment is configured to yield a melting temperature between 50°C to 75°C, such as between 50°C to 70°C. The MTDRs of a group of PTOs are preferably selected so their respective melting temperatures are easily detected and registered. Thus the MTDRs of a group of PTOs may differ in their respective melting temperatures by 2, 3, 4, 5, 6, 7, 8, 9, 10 or more degrees.

In an embodiment of the present disclosure step (a) comprises hybridizing the target nucleic acid sequence with a PTO; the PTO comprising (i) a targeting portion comprising a nucleotide sequence substantially complementary to the target nucleic acid sequence, and (ii) an MTDR, comprising a nucleotide sequence non-complementary to the target nucleic acid sequence, wherein the MDTR is configured to yield a melting temperature between 50°C to 75°C, such as between 50°C to 70°C, and (iii) at least one set of interactive labels comprising at least one fluorophore and at least one quencher; wherein the targeting portion of the PTO is configured to hybridize with the target nucleic acid sequence and the MTDR of the PTO is not configured to hybridize with the target nucleic acid sequence.

### Linker molecule

The PTO may further comprise a linker molecule, which is non-complementary to the target nucleic acid sequence and the CQO, between the targeting portion and the MTDR of the PTO. Thus in an embodiment of the present disclosure the PTO as described herein further comprises a linker molecule between the targeting portion and the MTDR of the PTO. In some embodiments of the present disclosure, the linker is a linker which is non-complementary to the target nucleic acid sequence and the CQO and wherein the linker molecule comprises 1-200 nucleotides, such as 1-50 nucleotides, such as 1-30 nucleotides, such as 2-20 nucleotides, such as about 4-14 nucleotides, such as 6-13 nucleotides, such as 8-12 nucleotides, such as 9-12 nucleotides, such as 11 nucleotides. The linker molecule may comprise or consist of non-nucleic acids such as non-natural or other organic compounds such as carbon chains such as C1-C40 alkanes such as a C6 carbon chain. In an embodiment of the present disclosure the linker comprises a mixture of nucleic acids and non-nucleic acids. In another embodiment of the present disclosure the linker comprises any organic compound. The linker may be a glycol linker, or any linker known to the person skilled in the art. An advantage of a non-nucleic acid may be that such linkers stop progression of the polymerase along the PTO.

### Blocking group

Preferably, the 3'-end of the PTO and/or the CQO is "blocked" to prohibit its extension during the PCR reaction. The blocking may be achieved in accordance with conventional methods. For instance, the blocking may be performed by adding to the 3'-hydroxyl group of the last nucleotide a chemical moiety such as biotin, a phosphate group, alkyl group, non-nucleotide linker, phosphorothioate and/or an alkane-diol. Alternatively, the blocking may be carried out by removing the 3'-hydroxyl group of the last nucleotide or using a nucleotide with no 3'-hydroxyl group such as dideoxynucleotide. Thus in an embodiment of the present disclosure the PTO and/or CQO further comprises a blocking group in the 3' end. In another embodiment of the present disclosure, said blocking group is selected from the group consisting of biotin, a phosphate group, alkyl group, non-nucleotide linker, a phosphorothioate, and/or an alkane-diol. In another embodiment of the present disclosure extension of the 3' end of the PTO and/or CQO is prohibited by removing the 3'-hydroxyl group of the last nucleotide of the PTO and/or CQO or by using a nucleotide with no 3'-hydroxyl group such as dideoxynucleotide. The quencher on the CQO may act as a blocking group. In an embodiment of the present disclosure said blocking group on the CQO is a quencher. In another embodiment of the present disclosure said blocking group on the CQO is a quencher located in the 3' end of said CQO.

The PTOs may be synthesized by click chemistry. A specific MTDR may be used for multiple assays whereas the targeting portion of the PTO varies dependent on the target to be measured. These two elements and the optional linker may thus be joined by click chemistry as is known to those skilled in the art; see also Nucleic Acids Symp Ser (2008) 52(1): 47-48.

### CQO

In an embodiment of the present disclosure each CQO is used in the present method in the determination of at least one, such as at least two, three, four, five, six, seven, eight, or nine, such as at least ten target nucleic acid sequences, such as 15, 20, 25 , 30, 35, 40, 45, 50 or more than 50 target nucleic acids. In a further embodiment of the present disclosure two CQOs are used to identify a multitude of target sequences wherein each CQO is used to identify at least one, such as at least two, three, four, five, such as at least ten or more target nucleic acid sequences. In a further embodiment of the present disclosure three CQOs, such as at least four, five, six, seven, such as at least eight CQOs are used to identify a multitude of target sequences wherein each CQO is used to identify at least one, such as at least two, three, four, five, such as at least ten target nucleic acid sequences.

In an embodiment of the present disclosure a CQO and optionally an enzyme having nuclease activity are in a liquid suspension or liquid solution. In an embodiment of the present disclosure at least one CQO and optionally the enzyme having nuclease activity are in a liquid suspension or liquid solution which is ready to use. By ready to use is implied that all conditions for running a PCR reaction are met, i.e. the required salts, pH and so forth are present. In an embodiment of the present disclosure at least one CQO and optionally the enzyme having nuclease activity are in a ready to use pellet. In an embodiment of the present disclosure at least one PTO and at least one CQO and optionally the enzyme having nuclease activity of the present disclosure are in a liquid suspension or liquid solution. In an embodiment of the present disclosure the at least one PTO and the at least one CQO and optionally an enzyme having nuclease activity of the present disclosure are in a liquid suspension or liquid solution which is ready to use. In a further embodiment of the present disclosure the at least one PTO and the at least one CQO and optionally the enzyme having nuclease activity are in a ready to use pellet. In an embodiment of the present disclosure the ready to use pellet comprises a substantially water free composition including e.g. salts and/or nucleotides for running a PCR reaction.

In another embodiment of the present disclosure the CQO comprises non-nucleic acid molecules.

In an embodiment the present disclosure relates to an oligonucleotide i.e. a CQO comprising at least one quencher and a capturing portion, wherein the capturing portion of the CQO is configured to hybridize to at least one, such as two or more PTOs of the present disclosure, wherein a single CQO may be used in the detection of a multitude of target nucleic acids sequences. A CQO hybridizes to the MTDR region of a PTO. In an embodiment of the present disclosure the CQO does not hybridize to the targeting portion of the PTO and/or to the optional linker of the PTO.

The CQO is for use according to the present assays.

### PTO and CQO fusion

The PTO and the CQO may be linked and configured to reversibly form a hairpin structure wherein the MTDR of the PTO and the capturing portion of the CQO hybridize and thus yield the hairpin structure. Figure 8a illustrates an example of how the PTO and CQO may yield a hairpin structure. In an embodiment of the present disclosure the PTO and the CQO are present on the same oligonucleotide. In another embodiment of the present disclosure the PTO and the CQO are linked. In another embodiment of the present disclosure the PTO and the CQO are situated on the same oligonucleotide, wherein the MTDR of the PTO and the capturing portion of the CQO are configured to reversibly form a hairpin structure.

The total length of each of the PTO and/or the CQO may vary. In one embodiment of the present disclosure the total length of the PTO is between 10 and 500 nucleotides, such as between 20 and 100, such as between 30 and 70 nucleotides. In another embodiment of the present disclosure the total length of the CQO is between 10 and 500 nucleotides or base pairs, such as between 15 and 100, such as between 20 and 50 nucleotides or base pairs. In the event the PTO and CQO are fused or linked the total length of the fusion product may be between 20 and 600 nucleotides. In another embodiment of the present disclosure the PTO and/or CQO comprises non-natural bases. Examples of artificial nucleic acids (or Xeno Nucleic Acids, XNA) include but are not limited to PNA, LNA, GNA and TNA. These compounds and their use are known to the person of skill. Specific examples of non-natural bases also include but are not limited to the following bases in base pair combinations: iso-C/iso-G, iso-dC/iso-dG. In another embodiment of the present disclosure the PTO and/or CQO comprises non-nucleic acid molecules.

### Fluorophores and quenchers

The inventors have shown that the distance between the fluorophore(s) located on the PTO and the quencher(s) located on the CQO affect the background melting curve generation. In one embodiment of the present disclosure the distance between the PTO fluorophore and the CQO quencher molecule is between 6 and 60 base pairs, such as between 10 to 35 base pairs, such as 15-25 base pairs. In another embodiment of the present disclosure the fluorophore of the PTO and the quencher (the closest quencher) of the CQO are separated by a distance of between 1 and 40 nucleotides or base pairs, such as between 6 to 35, 10 to 30, 15 to 25, such as about 18 nucleotides.

The distance between the at least one fluorophore of the PTO and the closest of the at least one quencher of the CQO is preferably such that the quenching is sufficient to allow differentiation of the signal between an activated Tag Duplex and a melted Tag Duplex where the PTO and the CQO are not hybridized and the signal is not quenched. Thus, some background signal may occur provided that this signal is lower than the true positive signal, thereby allowing discrimination between background signal and positive signal. Likewise, the choice of fluorophore and quencher should also be such that the background signal and positive signal can be discriminated, as the skilled person is aware.

In an embodiment step (b) of the present disclosure comprises hybridizing the PTO and the CQO; wherein the CQO comprises (i) a capturing portion comprising a nucleotide sequence which is reverse complementary to the MTDR of the PTO and (ii) at least one quenching molecule; wherein the MTDR of the PTO is configured to hybridize with the capturing portion of the CQO to form a Tag Duplex, wherein at least one fluorophore of the PTO and the at least one quencher of the CQO are separated by a distance of between 1 and 40 nucleotides or basepairs, such as between 6 to 35, 10 to 30, 15 to 25, such as about 20 nucleotides.

In an embodiment of the present disclosure the at least one set of interactive labels of the present disclosure comprises a fluorophore and a quencher, wherein the fluorescence emission from said fluorophore is quenched by said quencher. A set of interactive labels is configured to have compatible fluorophores and quenchers. In another embodiment of the present disclosure the at least one set of interactive labels comprises one, two, three, four, five, six, seven, or more sets of interactive labels. In a further embodiment of the present disclosure at least two groups of PTOs and CQOs are used for detection of at least two target nucleic acid sequences, wherein each group of PTOs and group of CQOs are configured to have compatible fluorophores and quenchers. In another embodiment of the present disclosure the main interaction between the at least one set of interactive labels is mediated by fluorescence resonance energy transfer (FRET).

The inventors have also shown that the distance between the interactive set of labels of the PTO comprising at least one fluorophore and at least one quencher also affects the undesirable background melting curve generation. In an embodiment of the present disclosure the interactive set of labels of the PTO is separated by a distance between 1 and 40 nucleotides or base pair, such as between 6 and 35, 10 - 30, 15 to 25, such as about 20 nucleotides. In an embodiment of the present disclosure step (a) of the method described herein comprises hybridizing the target nucleic acid sequence with a PTO; the PTO comprising (i) a targeting portion comprising a nucleotide sequence substantially complementary to the target nucleic acid sequence, and (ii) a MTDR, comprising a nucleotide sequence non-complementary to the target nucleic acid sequence, and (iii) at least one set of interactive labels comprising at least one fluorophore and at least one quencher, wherein the at least one fluorophore and at least one quencher are separated by a distance between 3.4Å and 136Å, such as between 20.4 Å and 119Å, 34Å and 102Å, 51Å and 85Å, such as about 61.2Å ; wherein the targeting portion of the PTO is configured to hybridize with the target nucleic acid sequence and MTDR of the PTO is not configured to hybridize with the target nucleic acid sequence. In an embodiment of the present disclosure step (a) of the method described herein comprises hybridizing the target nucleic acid sequence with a PTO; the PTO comprising (i) a targeting portion comprising a nucleotide sequence substantially complementary to the target nucleic acid sequence, and (ii) a MTDR, comprising a nucleotide sequence non-complementary to the target nucleic acid sequence, and (iii) at least one set of interactive labels comprising at least one fluorophore and at least one quencher, wherein the at least one fluorophore and at least one quencher are separated by a distance between 1 and 40 nucleotides or base pair, such as between 6 and 35, 10 - 30, 15 to 25, such as about 18 nucleotides; wherein the targeting portion of the PTO is configured to hybridize with the target nucleic acid sequence and MTDR of the PTO is not configured to hybridize with the target nucleic acid sequence.

In an embodiment of the present disclosure the interactive set of labels of the PTO are placed so emission from the at least one fluorophore of the PTO is quenched by the at least one quencher of the PTO and by the at least one quencher of the CQO when the PTO and CQO are hybridized. In another embodiment of the present disclosure the interactive set of labels of the PTO are placed so emission from the at least one fluorophore of the PTO is quenched by the at least one quencher of the PTO and by the at least one quencher of the CQO, wherein the level of quenching of the at least one fluorophore of the PTO by the least one quencher of the PTO and the at least one quencher of the CQO is substantially similar when the PTO and CQO are hybridized. In a preferred embodiment of the present disclosure the interactive set of labels of the PTO are placed so emission from the at least one fluorophore of the PTO is quenched by the at least one quencher of the PTO and by the at least one quencher of the CQO, wherein the distance between of the at least one fluorophore of the PTO by the least one quencher of the PTO and the at least one quencher of the CQO is substantially similar when the PTO and CQO are hybridized. In a further embodiment of the present disclosure the interactive set of labels of the PTO are placed so the level of quenching of the at least one fluorophore of the PTO by the at least one quencher of the PTO is substantially similar to and/or stronger than the level of quenching by the at least one quencher of the CQO when the PTO and CQO are hybridized. In another embodiment of the present disclosure the interactive set of labels of the PTO are placed so the distance from the at least one fluorophore of the PTO and the at least one quencher of the PTO is substantially similar to and/or shorter than the distance from the at least one fluorophore of the PTO to the at least one quencher of the CQO when the PTO and CQO are hybridized.

Fluorophores which may be conjugated to an oligonucleotide may be used in the present disclosure. In an embodiment of the present disclosure, the PTO described herein comprises at least one fluorophore. In another embodiment of the present disclosure the at least one fluorophore of the PTO is selected from the group comprising 6-carboxyfluorescein (FAM), tetrachlorofluorescein (TET) or a combination hereof. In another embodiment of the present disclosure the PTO of the present disclosure comprises more than one fluorophore, such as two, three, four, five, six, seven, and/or such as eight fluorophores. In an embodiment of the present disclosure the PTO of the present disclosure comprises two or more identical fluorophores, such as three, four, five, six, seven, and/or such as eight identical fluorophores. In another embodiment of the present disclosure the PTO of the present disclosure comprises two or more different fluorophores, such as three, four, five, six, seven, and/or such as eight different fluorophores.

To facilitate quenching of the at least one fluorophore on the PTO as described herein the PTO comprises at least one quencher molecule. Quenchers which may be conjugated to an oligonucleotide may be used in the present disclosure. The at least one quencher and the at least one fluorophore of the PTO are configured to be at least one set of interactive labels. In an embodiment of the present disclosure the PTO described herein comprises at least one quencher. In another embodiment of the present disclosure the at least one quencher of the PTO is configured to quench the at least one fluorophore of the PTO. In another embodiment of the present disclosure the at least one fluorophore of the PTO is selected from the group comprising black hole quencher (BHQ) 1, BHQ2, and BHQ3, Cosmic Quencher (e.g. from Biosearch Technologies, Novato, USA), Excellent Bioneer Quencher (EBQ) (e.g. from Bioneer, Daejeon, Korea) or a combination hereof. In a further embodiment of the present disclosure the PTO of the present disclosure comprises more than one quencher, such as two, three, four, five, six, seven, and/or such as eight quenchers. In an embodiment of the present disclosure the PTO of the present disclosure comprises two or more identical quenchers, such as three, four, five, six, seven, and/or such as eight identical quenchers. In another embodiment of the present disclosure the PTO of the present disclosure comprises two or more different quenchers, such as three, four, five, six, seven, and/or such as eight different fluorophores.

To facilitate quenching of the at least one fluorophore on the PTO as described herein the CQO comprises at least one quencher molecule. Most quenchers which may be conjugated to an oligonucleotide may be used in the present disclosure. However, the at least one quencher of the CQO and the at least one fluorophore of the PTO may be configured to be at least one set of interactive labels. In an embodiment of the present disclosure the CQO described herein comprises at least one quencher. In another embodiment of the present disclosure the at least one quencher of the CQO is configured to quench the at least one fluorophore of the PTO as described herein. In another embodiment of the present disclosure the at least one quencher of the CQO is selected from the group comprising black hole quencher (BHQ) 1, BHQ2, and BHQ3 (from Biosearch Technologies, Novato, USA). In a further embodiment of the present disclosure the CQO of the present disclosure comprises more than one quencher, such as two, three, four, five, six, seven, and/or such as eight quenchers. In an embodiment of the present disclosure the CQO of the present disclosure comprises two or more identical quenchers, such as three, four, five, six, seven, and/or such as eight quenchers. In another embodiment of the present disclosure the CQO of the present disclosure comprises two or more different quenchers, such as three, four, five, six, seven, and/or such as eight fluorophores.

A fluorophore which may be useful in the present disclosure may include any fluorescent molecule known in the art. Examples of fluorophores are: Cy2™ Cfflfi), YO-PRn™-1 (509), YDY0™-1 (509), Calrein (517), FITC (518), FluorX™ (519), Alexa™ (520), Rhodamine 110 (520), Oregon Green™ 500 (522), Oregon Green™ 488 (524), RiboGreen™ (525), Rhodamine Green™ (527), Rhodamine 123 (529), Magnesium Green™(531), Calcium Green™ (533), TO-PRO™-I (533), TOTOI (533), JOE (548), BODIPY530/550 (550), Dil (565), BODIPY TMR (568), BODIPY558/568 (568), BODIPY564/570 (570), Cy3™ (570), Alexa™ 546 (570), TRITC (572), Magnesium Orange™ (575), Phycoerythrin R&B (575), Rhodamine Phalloidin (575), Calcium Orange™(576), Pyronin Y (580), Rhodamine B (580), TAMRA (582), Rhodamine Red™ (590), Cy3.5(TM) (596), ROX (608), Calcium Crimson™ (615), Alexa™ 594 (615), Texas Red(615), Nile Red (628), YO-PRO™-3 (631), YOYO™-3 (631), R-phycocyanin (642), C-Phycocyanin (648), TO-PRO™-3 (660), TOT03 (660), DiD DiIC(5) (665), Cy5™ (670), Thiadicarbocyanine (671), Cy5.5 (694), HEX (556), TET (536), Biosearch Blue (447), CAL Fluor Gold 540 (544), CAL Fluor Orange 560 (559), CAL Fluor Red 590 (591), CAL Fluor Red 610 (610), CAL Fluor Red 635 (637), FAM (520), Fluorescein (520), Fluorescein-C3 (520), Pulsar 650 (566), Quasar 570 (667), Quasar 670 (705) and Quasar 705 (610). The number in parenthesis is a maximum emission wavelength in nanometers. In a preferred embodiment t of the present disclosure he fluorophore is selected from the group consisting of FAM and/or TET. It is noteworthy that a non-fluorescent black quencher molecule capable of quenching a fluorescence of a wide range of wavelengths or a specific wavelength may be used in the present disclosure. In a preferred embodiment of the present disclosure the set of interactive labels are FAM/BHQ. Other suitable pairs of fluorophores/quenchers are known in the art.

### Step (a) Hybridizing the PTO to a target sequence

Step (a) of the present disclosure relates hybridization of the PTO of the present disclosure to the target nucleic acid. In an embodiment of the present disclosure step (a) of the method described herein relates to hybridizing the target nucleic acid sequence with a PTO; the PTO comprising (i) a targeting portion comprising a nucleotide sequence substantially complementary to the target nucleic acid sequence, and (ii) a MTDR, comprising a nucleotide sequence non-complementary to the target nucleic acid sequence, and (iii) at least one set of interactive labels comprising at least one fluorophore and at least one quencher; wherein the targeting portion of the PTO is configured to hybridize with the target nucleic acid sequence and MTDR of the PTO is not configured to hybridize with the target nucleic acid sequence.

In an embodiment of the present disclosure step (a) comprises hybridizing the target nucleic acid sequence with a PTO; the PTO comprising (i) a targeting portion comprising a nucleotide sequence substantially complementary to the target nucleic acid sequence, and (ii) a MTDR, comprising a nucleotide sequence non-complementary to the target nucleic acid sequence, and (iii) at least one set of interactive labels comprising at least one fluorophore and at least one quencher; wherein the targeting portion of the PTO is configured to hybridize with the target nucleic acid sequence and MTDR of the PTO is not configured to hybridize with the target nucleic acid sequence, wherein the PTO is between 10 and 500 nucleotides, such as between 20 and 100, such as between 30 and 70 nucleotides or base pairs. The PTOs and CQOs of the present disclosure may be premixed prior to addition of the target sequence in the present disclosure. Thus the Tag Duplex formation of step (b) of the present disclosure may form prior to hybridization of the PTO to the target sequence. In an embodiment of the present disclosure step (b) is performed prior to step (a) as follows;
(b) hybridizing a PTO with a CQO (Capturing and Quenching Oligonucleotide), wherein the PTO comprises (i) a targeting portion comprising a nucleotide sequence substantially complementary to the target nucleic acid sequence, and (ii) a Melting Temperature Deciding Region (MTDR), comprising a nucleotide sequence non-complementary to the target nucleic acid sequence, and (iii) at least one set of interactive labels comprising at least one fluorophore and at least one quencher; wherein the targeting portion of the PTO is configured to hybridize with the target nucleic acid sequence and MTDR of the PTO is not configured to hybridize with the target nucleic acid sequence; ; wherein the CQO comprises (i) a capturing portion comprising a nucleotide sequence which is reverse complementary to the MTDR of the PTO and (ii) at least one quenching molecule; wherein the MTDR of the PTO is configured to hybridize with the capturing portion of the CQO to form a Tag Duplex; and
(a) hybridizing the target nucleic acid sequence with said Tag Duplex;
(c) contacting the Tag Duplex with an enzyme having nuclease activity; wherein the enzyme having nuclease activity induces cleavage of the Tag Duplex when the Tag Duplex is hybridized with the target nucleic acid sequence thereby releasing an activated Tag Duplex fragment comprising a PTO fragment comprising the MTDR hybridized to the capturing portion of the CQO and the at least one fluorophore;
(d) melting and/or hybridizing said activated Tag Duplex fragment to obtain a signal from the at least one fluorophore, and
(e) detecting the activated Tag Duplex fragment by measuring the signal from the at least one fluorophore; wherein the signal is indicative of the presence of the target nucleic acid sequence in the nucleic acid mixture.

It will be understood that in order to obtain a signal from the at least one fluorophore, said activated Tag Duplex fragment does not comprise the at least one quencher comprised in the at least one set of interactive labels of the MTDR. In other words, the signal is obtained when the at least one fluorophore and the at least one quencher of the at least one set of interactive labels no longer interact.

The PTOs, CQOs and target sequences of the present disclosure may also be mixed simultaneously. In an embodiment of the present disclosure step (a) and step (b) of the present disclosure may be carried out in any order or simultaneously.

### Upstream and downstream oligonucleotides

The addition of a pair of PCR primers located upstream and downstream of the binding site of the PTO to the target nucleic acid sequence increases the specificity of PCR assays and assists in avoiding false positive hybridization signals. Thus the present disclosure may further include an upstream primer which is complementary to the target nucleic acid and a downstream primer which may hybridize downstream of the PTO binding site on the target nucleic acid. The upstream and downstream oligonucleotides are configured not to overlap with the PTO binding site of the target nucleic acid sequence. The upstream and downstream primers may be located within 2000 base pairs of the target nucleic acid sequence. In one embodiment of the present disclosure the upstream oligonucleotide and downstream nucleotide are located at least one base pair from the PTO binding site of the target nucleic acid sequence. In another embodiment of the present disclosure the upstream and/or downstream oligonucleotides are located between 1 - 2000 base pairs from the PTO binding site of the target nucleic acid sequence. The upstream and/or downstream oligonucleotides may be located more than 2000 base pairs (2 kb) from the target nucleic acid sequence, such as 2.5 kb, such as 3 kb, such as 3.5 kb, such as 4 kb, such as 5 kb, such as 10 kb, such as 20 kb from the target nucleic acid sequence.

### Decontamination

Decontamination of the reaction vessel may take place prior to step (a). Thus in an embodiment of the present disclosure a UNG treatment step (BioTechniques 38:569-575 (April 2005)) and/or a denaturation step is used prior to step (a). RNA decontamination treatments known to the skilled person may be applied. In an embodiment of the present disclosure a decontamination treatment step and/or a denaturation step is used prior to step (a).

### Step (b) Hybridizing a CQO to the PTO forming a Tag Duplex

Step (b) of the present method concerns the hybridization of the PTO and the CQO which forms a Tag Duplex. In an embodiment of the present disclosure step (b) of the present method comprises hybridizing the PTO and the CQO; wherein the CQO comprises (i) a capturing portion comprising a nucleotide sequence which is reverse complementary to the MTDR of the PTO and (ii) at least one quenching molecule; wherein the MTDR of the PTO is configured to hybridize with the capturing portion of the CQO to form a Tag Duplex.

As previously described the inventors have shown that the distance between the fluorophore(s) located on the PTO and the quencher(s) located on the CQO affect the background melting curve generation.

In an embodiment of the present disclosure step (b) of the present method comprises hybridizing the PTO and the CQO; wherein the CQO comprises (i) a capturing portion comprising a nucleotide sequence which is reverse complementary to the MTDR of the PTO and (ii) at least one quenching molecule; wherein the MTDR of the PTO is configured to hybridize with the capturing portion of the CQO to form a Tag Duplex, wherein the distance between the at least one fluorophore on the PTO and the at least one quenching molecule on the CQO quencher molecule is between 1 and 60 base pairs, such as between 10 to 35 base pairs, such as 15-25 base pairs, such as about 18 base pairs.

In an embodiment of the present disclosure step (b) of the present method comprises hybridizing the PTO and the CQO; wherein the CQO comprises (i) a capturing portion comprising a nucleotide sequence which is reverse complementary to the MTDR of the PTO and (ii) at least one quenching molecule configured for quenching of the at least one fluorophore of the PTO; wherein the MTDR of the PTO is configured to hybridize with the capturing portion of the CQO to form a Tag Duplex.

### Step (c) Release of an activated Tag Duplex fragment

### Step (c) of the present disclosure relates to nuclease mediated cleavage of the Tag

Duplex which forms a released Tag Duplex fragment. In an embodiment of the present disclosure step (c) of the present disclosure comprises contacting the Tag Duplex from step (b) with an enzyme having nuclease activity; wherein the enzyme having nuclease activity induces cleavage of the Tag Duplex when the Tag Duplex is hybridized with the target nucleic acid sequence thereby releasing an activated Tag Duplex fragment comprising a PTO fragment comprising the MTDR hybridized to the capturing portion of the CQO and the at least one fluorophore.

To avoid false positive signals the at least one quencher of the CQO is configured to reversibly quench the at least one fluorophore of the PTO when the activated Tag Duplex fragment is hybridized. In an embodiment of the present disclosure of the present method the at least one quencher of the CQO is configured to reversibly quench the at least one fluorophore of the activated Tag Duplex fragment. As explained above, the quencher of the CQO and the fluorophore of the PTO should be chosen such that the background signal and the positive signal can be discriminated. Likewise, the distance between the quencher of the CQO and the fluorophore of the PTO may have to be optimized to obtain a desired discrimination of signals.

Any enzyme having nuclease activity may induce the release of the Tag Duplex as shown in figure 1. In an embodiment of the present disclosure the nuclease activity described above is 5' nuclease activity of a FEN nuclease (flap endonuclease). In another embodiment of the present disclosure the enzyme having nuclease activity is a template dependent DNA polymerase such as a thermostable template dependent DNA polymerase, such as a Taq polymerase and/or a Sso7d-fusion polymerase or mixtures thereof. In a preferred embodiment of the present disclosure the enzyme having nuclease activity is a Sso7d-fusion polymerase. In another preferred embodiment of the present disclosure the enzyme having nuclease activity is the GoTaq polymerase. In another embodiment of the present disclosure the enzyme having nuclease activity is a template dependent DNA polymerase having 5' to 3' exonuclease activity. Thus in an embodiment of the present disclosure the nuclease is an exonuclease.

The skilled person knows that the concentration of polymerase may influence its activity. The optimal concentration may also depend on further parameters such as the concentration of target, template, or the sequence of the nucleic acids present in the reaction. The skilled person knows how to optimize the polymerase concentration in order to achieve good results.

The release of the Tag Duplex, resulting in the formation of the activated Tag Duplex fragment is mediated by the enzyme having nuclease activity upon extension of the upstream oligonucleotide described herein. In an embodiment of the present disclosure the cleavage of the PTO part of the Tag Duplex is induced by said template dependent DNA polymerase extending the upstream oligonucleotide, wherein said polymerase has 5' nuclease activity. In another embodiment of the present disclosure the cleavage of the PTO part of the Tag Duplex is induced by said template dependent DNA polymerase upon extension of the upstream oligonucleotide, wherein said polymerase has 5' nuclease activity.

Tag Duplex fragment is released when the part of the 5' targeting portion of the PTO is cleaved. The cleavage results in a reduced affinity between the targeting portion of the PTO and the target nucleic acid sequence which consequently results in dissociation of the target nucleic acid sequence and the Tag Duplex thereby forming the activated Tag Duplex fragment. In an embodiment of the present disclosure the at least one quencher on the PTO as described herein is released from the PTO by the enzyme having nuclease activity. In another embodiment of the present disclosure the enzyme having nuclease activity removes the at least one quencher of the PTO part of the activated Tag Duplex fragment. In another embodiment of the present disclosure the activated Tag Duplex fragment comprises a PTO fragment wherein the at least one quencher of the PTO is not present. The at least one quencher may not be present on the activated Tag Duplex fragment as a consequence of the nuclease activity of the enzyme having nuclease activity as described herein and illustrated on figure 1.

The presence of the activated Tag Duplex and/or activated PTO may be detected by qPCR and/or real time PCR. Preferably only activated Tag Duplex is detected by real time PCR.

### Step (d) Melting the activated Tag Duplex fragment

Step (d) of the present disclosure relates to the melting of the activated Tag Duplex fragment from step (c). In an embodiment of the present disclosure step (d) of the present disclosure comprises melting and/or hybridizing said activated Tag Duplex fragment to obtain a signal from the at least one fluorophore. The temperature at which the melting occurs is registered.

The melting may be carried out by conventional technologies, including, but not limited to, heating, alkali, formamide, urea and glycoxal treatment, enzymatic methods (e.g., helicase action), and binding proteins. For instance, the melting can be achieved by heating at temperature ranging from 30°C to 100 °C.

When the activated Tag Duplex fragment is heated over a range of temperatures the MTDR of the PTO dissociates from the CQO of the present disclosure. The temperature at which one half of an activated Tag Duplex fragment duplex will dissociate to become single stranded is determined by the stability Tag Duplex which is determined by the melting temperature region (MTDR). Thus in an embodiment of the present disclosure the activated Tag Duplex fragment I heated over a range of temperatures. In another embodiment of the present disclosure the activated Tag Duplex fragment is heated from 30°C to 100°C, such as from 35°C to 100°C, such as from 40°C to 75°C such as from 45°C to 70°C. In a preferred embodiment of the present disclosure the melting temperature of the activated Tag Duplex fragment is 45°C to 70°C. In another embodiment of the present disclosure the activated Tag Duplex fragment is melted at a predetermined temperature.

As long as the activated Tag Duplex fragment is double stranded the emission from the at least one fluorophore on the PTO is substantially quenched by the at least one quencher on the CQO. When the activated Tag Duplex fragment dissociates and become single stranded the emission of the at least one fluorophore on the PTO may be unquenched by the at least one quencher on the CQO. Thus in an embodiment of the present disclosure the emission from the at least one fluorophore is unquenched when the Tag Duplex is melted in step (d). In another embodiment of the present disclosure the emission from the at least one fluorophore is unquenched when the Tag Duplex dissociates and become single stranded in step (d). In another embodiment of the present disclosure the presence of an activated Tag Duplex fragment is determined by a melting curve analysis and/or a hybridization curve analysis. In a further embodiment of the present disclosure the presence of an activated Tag Duplex fragment is determined by a melting curve analysis and/or a hybridization curve analysis wherein the identified melting temperature of the activated Tag Duplex fragment is determined by the MTDR of the PTO described herein.

### Step (e) Detection of signal from the melted Tag Duplex fragment.

Step (e) of the present disclosure relates to detection of a signal as a consequence of the Tag Duplex melting in step (d). In an embodiment of the present disclosure of the present disclosure step (e) comprises detecting the activated Tag Duplex fragment by measuring the signal from the at least one fluorophore; wherein the signal is indicative of the presence of the target nucleic acid sequence in the nucleic acid mixture.

The nature of the signal to be measured is dependent on the at least one fluorophore on the PTO and may be determined by a range of analytical methods for example real-time PCR detection systems.

A melting curve or hybridization curve may be obtained by conventional technologies. For example, a melting curve or hybridization curve may comprise a graphic plot or display of the variation of the output signal with the parameter of hybridization stringency. The output signal may be plotted directly against the hybridization parameter. Typically, a melting curve or hybridization curve will have the output signal, for example fluorescence, which indicates the degree of duplex structure (i.e. the extent of hybridization), plotted on the Y-axis and the hybridization parameter on the X axis (i.e. the temperature).

For recording a melting curve by fluorescence, typically the overall sample temperature can be either increased or decreased stepwise with set equilibration time of 0-360 s at each temperature. Typically a step size between 0.5°C and 2°C is used but it could be lowered to 0.1 °C depending on the desired accuracy. At every temperature, a readout of the fluorescence is recorded for the relevant wavelength. Based on the melting curve, the TM of a DNA duplex under the conditions applied can be determined.

The method of choice for nucleic acid (DNA, RNA) quantification in all areas of molecular biology is real-time PCR or quantitative PCR (qPCR). The method is so-called because the amplification of DNA with a polymerase chain reaction (PCR) is monitored in real time (qPCR cyclers constantly scan qPCR plates).

Fluorescent reporter probes (Taqman probes or dual-labeled probes) detect only the DNA containing the sequence complementary to the probe; therefore, use of the reporter probe significantly increases specificity, and enables performing the technique even in the presence of other dsDNA. Using different-coloured labels, fluorescent probes can be used in multiplex assays for monitoring several target sequences in the same tube. The method relies on a DNA-based probe with a fluorescent reporter at one end and a quencher of fluorescence at the opposite end of the probe. The close proximity of the reporter to the quencher prevents detection of its fluorescence; breakdown of the probe by the 5' to 3' exonuclease activity of the Taq polymerase breaks the reporter-quencher proximity and thus allows unquenched emission of fluorescence, which can be detected after excitation with a laser. An increase in the product targeted by the reporter probe at each PCR cycle therefore causes a proportional increase in fluorescence due to the breakdown of the probe and release of the reporter. The PCR is prepared as normal and the reporter probe is added. As the reaction commences, during the annealing stage of the PCR both probe and primers anneal to the DNA target. Polymerisation of a new DNA strand is initiated from the primers, and once the polymerase reaches the probe, its 5'-3'-exonuclease degrades the probe, physically separating the fluorescent reporter from the quencher, resulting in an increase in fluorescence. Fluorescence is detected and measured in a real-time PCR machine, and its geometric increase corresponding to exponential increase of the product is used to determine the quantification cycle (Cq) in each reaction.

The signal can be measured either at the temperature of annealing or at the temperature of denaturation of the duplexes present in the reaction. Accordingly, the signal can be measured at a temperature of between 55 and 65°C, such as between 56 and 64°C, such as between 57 and 63°C, such as between 58 and 62°C, such as between 59 and 61 °C, such as at 60°C. In other embodiments of the present disclosure, the signal can be measured at a temperature of between 90 and 100°C, such as between 91 and 99°C, such as between 92 and 98°C, such as between 94 and 97°C, such as between 95 and 96°C, such as at 95°C. The skilled person knows how to determine which temperature provides the best readout signal.

As shown in example 5, the concentration of target influences the strength of the signal to be detected. Thus, target concentration may be adjusted to improve the signal if needed.

### Target nucleic acid sequences

The target nucleic acid sequence as used herein refers to any sequence which is desirable to identify in a mixture of nucleic acid sequences. The simple assay of the present disclosure has a multitude of applications. A non-exhaustive list of applications of the assay of the present disclosure may be:
- Human and/or veterinary diagnostics
- Food and/or feed quality and safety
- Environmental surveillance
- Scientific research

Thus in an embodiment of the present disclosure the target nucleic acid sequence of the present disclosure is from a pathogenic organism such as a bacterium, virus, fungus, and/or protozoan. In another embodiment of the present disclosure the target nucleic acid sequence of the present disclosure is from a pathogenic organism capable of infecting a farm animal such as a cow, chicken, pig, horse, sheep, and/or goat. In a preferred embodiment of the present disclosure the target nucleic acid sequence of the present disclosure is from a pathogenic organism capable of infecting a mammal such as a human being, cow, pig, horse, sheep, and/or goat. In a more preferred embodiment of the present disclosure the target nucleic acid sequence of the present disclosure is from a pathogenic organism capable of infecting a human being.

In an embodiment of the present disclosure the target nucleic acid sequence of the present disclosure is from a virus capable of infecting a human being. In a further embodiment of the present disclosure the target nucleic acid sequence of the present disclosure is from a virus capable of infecting a human being which causes a mortality rate higher than 10%. In an embodiment of the present disclosure the virus is an Ebola virus.

In an embodiment of the present disclosure the target nucleic acid sequence of the present disclosure is from bacteria capable of infecting a human being. In another embodiment of the present disclosure the target nucleic acid sequence of the present disclosure is from bacteria capable of infecting a human being which causes a mortality rate higher than 10%.

In an embodiment of the present disclosure the target nucleic acid sequence of the present disclosure is from a pathogenic organism causing a sexually transmitted disease selected from the group consisting of Chlamydia, Gonorrhea, and Herpes.

In an embodiment of the present disclosure the target nucleic acid sequence of the present disclosure is from a pathogenic organism selected from the group comprising Methicillin Resistant Staphylococcus Aureus (MRSA).

### Kit for detection of target nucleic acid sequences

The elements the present disclosure may be comprised within a kit of parts.

Thus an aspect of the present disclosure relates to a kit of parts for detection of a target nucleic acid sequence, the kit comprising:
i. optionally at least one PTO as described herein, and
ii. at least one CQO described herein, and
iii. optionally an enzyme having nuclease activity, and
iv. optionally instructions on how to detect a target nucleic acid sequence.

The kit may be used for detection of more than one target nucleic acid sequences. In an embodiment of the present disclosure the kit described herein further comprises:
i. optionally at least two PTOs from at least two different groups of PTOs, and
ii. at least two CQOs.

The kit may also contain at least one downstream and/or upstream oligonucleotide as described herein. In an embodiment of the present disclosure the kit further comprises a downstream oligonucleotide and/or an upstream oligonucleotide as described herein.

Additionally the kit may further comprise an enzyme with nuclease activity. Thus in an embodiment of the present disclosure the kit further comprises an enzyme with nuclease activity as described herein.

In an embodiment of the present disclosure the kit described herein is a liquid suspension or liquid solution. In an embodiment of the present disclosure the kit described above is a liquid suspension or liquid solution which is ready to use. In a further embodiment of the present disclosure the described kit is in a ready to use pellet. In an embodiment of the present disclosure the ready to use pellet comprises a substantially water free composition comprising i), ii), and iii) of said kit.

In an embodiment of the present disclosure the kit described herein comprises PTOs and CQOs which are partially and/or fully hybridized.

The kit may also include at least one Tag Duplex which may be used as control and for Tₘ calibration of e.g. the applied analytical equipment. The Tₘ of an oligonucleotide may vary depending on e.g. the salt concentration, DNA concentration, pH and the presence of denaturants (such as formamide or DMSO). Such control may be desirable if the samples to be analyzed contain varying i.e. salt concentrations. In an embodiment of the present disclosure the kit described herein further comprises a control sample and/or control Tag Duplex.

### Diagnosis

The present methods may be used in the diagnosis and/or treatment of individuals in need thereof.

Thus an embodiment of the present disclosure relates to a method of diagnosing an individual as having a disease or disorder characterized by the presence of a target nucleic acid sequence said method comprising the steps of the assay as indicated elsewhere and resulting in the detection of said target nucleic acid sequence.

A non-limiting example hereof is: A method of diagnosing an individual as having a disease or disorder characterized by the presence of a target nucleic acid sequence said method comprising the steps of:
Step (a) hybridizing a target nucleic acid sequence with a PTO (Probing and Tagging Oligonucleotide); the PTO comprising (i) a targeting portion comprising a nucleotide sequence substantially complementary to the target nucleic acid sequence, and (ii) a Melting Temperature Deciding Region (MTDR), comprising a nucleotide sequence non-complementary to the target nucleic acid sequence, and (iii) at least one set of interactive labels comprising at least one fluorophore and at least one quencher;
Step (b) hybridizing said PTO with a CQO (Capturing and Quenching Oligonucleotide); wherein the CQO comprises (i) a capturing portion comprising a nucleotide sequence which is reverse complementary to the MTDR of the PTO and (ii) at least one quenching molecule; wherein the MTDR of the PTO is configured to hybridize with the capturing portion of the CQO to form a Tag Duplex;
Step (c) contacting the Tag Duplex with an enzyme having nuclease activity; wherein the enzyme having nuclease activity induces cleavage of the Tag Duplex when the Tag Duplex is hybridized with the target nucleic acid sequence thereby releasing an activated Tag Duplex fragment comprising a PTO fragment comprising the MTDR hybridized to the capturing portion of the CQO and the at least one fluorophore;
Step (d) melting and/or hybridizing said activated Tag Duplex fragment to obtain a signal from the at least one fluorophore, and
Step (e) detecting the activated Tag Duplex fragment by measuring the signal from the at least one fluorophore; wherein the signal is indicative of the presence of the target nucleic acid sequence and thus the presence of the disease or disorder in said individual.

The disease or disorder may be an infection caused i.e. by a pathogen or be a genetic disorder or disease.

### Reaction mixture

In a further embodiment the disclosure comprises a reaction mixture. Thus an embodiment of the disclosure provides a reaction mixture for use in a process for the amplification and/or detection of a target nucleic acid sequence in a sample wherein the reaction mixture, prior to amplification, comprises at least one pair of oligonucleotide primers, at least one PTO and at least one CQO, wherein said pair of primers, PTO and CQO are characterized in that said pair of oligonucleotide primers comprises a first a primer complementary to said target nucleic acid and which primes the synthesis of a first extension product that is complementary to said target nucleic acid, and a second primer complementary to said first extension product and which primes the synthesis of a second extension product; and said PTO hybridizes to a nucleotide sequence substantially complementary to the target nucleic acid sequence or the complement of said target nucleic acid, wherein said region is between one member of said primer pair and the complement of the other member of said primer pair and the PTO comprises at least one set of interactive labels, a MTDR, and optionally a linker between the targeting portion and the MTDR; and wherein the CQO comprises at least one quencher and a capturing portion, said capturing portion being configured to hybridize to the PTO. The reaction mixture may comprise several oligonucleotide primer pairs, several PTOs and a single CQO. The reaction mixture may comprise a single CQO configured to hybridize to all PTOs in the reaction mixture.

### Computer implemented method

Another aspect of the present disclosure relates to a computer-implemented method for identifying at least one target sequence, the method comprising the steps of 1) providing information about PTOs, CQOs, target sequences, and 2) obtaining signals from at least one melted Tag Duplex fragment, and 3) identification of at least one target sequence on the basis of said provided information and obtained signals.

### Examples

### Example 1 Distance between the fluorophore and the CQO quencher

The following example shows results of a PCR reaction comprising 5 different designs of tagging probes and a TaqMan probe specific for the *ipaH* gene. All reactions are performed with the 2 common primers (DEC229F and DEC230R). Tagging probes were designed such that FAM is inserted furthest from the location of quenchers in the hybridised targeting portion of the PTO in DEC486P and closest to the location of quenchers in the hybridized targeting portion of the PTO in DEC490P. The five different tagging probe designs were tested in combination with a single CQO design (DEC481rP). The results illustrates that by increasing the distance between FAM and the location of quenchers in the hybridized targeting portion of the PTO, background melting curve generation is reduced. It further illustrates that the preferred PTO design (DEC486P) generates PCR amplification curves only in the presence of the specific target, and also only generates melting curve signal in the presence of amplified target. The other designs included (DEC487P-490P) all show background melting curves in the NTC reaction indicating false positive results. The TaqMan probe DEC464 is included as a positive control for the PCR reaction.

**Table 1: Primer and probes sequences (5' to 3'):**

| **PCR Primers:** | | **Seq ID NO:** |
|---|---|---|
| DEC229F | ZGTCCATCAGGCATCAGAAGG | 1 |
| DEC230R | ZGGTAGACTTCTATCTCATCCAC | 2 |

| CQO **(Quenching probe)** | | |
|---|---|---|
| DEC481 rP | | 3 |

| **Tagging Probe (PTO)** | | |
|---|---|---|
| DEC486P | | 4 |
| DEC487P | | 5 |
| DEC488P | | 6 |
| DEC489P | | 7 |
| DEC490P | | 8 |

| **TaqMan Probe** | | |
|---|---|---|
| DEC464 | FAM-AA TGTTCCGCCTCGAAA TTCTGGAG-BHQ1 | 9 |

| | | |
|---|---|---|
| Z= TINA, BHQ1= black hole quencher 1, BHQ2= black hole quencher 2, BHQ3= black hole quencher 3, (dT-FAM)= internal FAM attached to T. (dT-BHQ1)=internal BHQ1 attached to T. (dT-BHQ2)=internal BHQ2 attached to T. | | |

### PCR reaction:

A 10 µL reaction was prepared that contained 1x SsoAdvanced Universal Probes Supermix (prod. #172-5280, Bio-Rad), 200 nM forward and reverse primer, 400 nM tagging probe, 800 nM quenching probe, 1:200 dilution of target, 0,25 U Uracil DNA Glycosylase, 1 U/µL, (#EN0361 Fermentas). Target was prepared by mixing a single colony of E. coli in 200 ul water and boiling 95°C for 15 minutes. 25 µL boiled target was subsequently added to 100 µL sterile water as a target stock solution which was diluted 5x in the final PCR reaction. Reactions were assembled in AB gene SuperPlate 96-well PCR plate (kat.nr. AB2800) and sealed with Optically clear, adhesive, Microseal B film (Bio-Rad, Cat.nr. MSB1001).

PCR reaction mix was subjected to the following PCR cycling and melting curve program (Bio-Rad CFX96 Real-Time PCR Detection System):

### PCR program:

| | **Temp (°C)** | | **Time** | | |
|---|---|---|---|---|---|
| **1** | 40 | for | 10 | minutes | UNG treatment |
| **2** | 95 | for | 10 | minutes | Activation/denaturation |
| **3** | 95 | for | 15 | seconds | a) Denaturation |

| **Plate Read** | | | | | |
|---|---|---|---|---|---|
| **4** | **60** | for | 60 | seconds | b) Annealing/elongation |
| | GoTo 3, 39 | more tim | mes | | |
| **5** | 95 | for | 10 | seconds | |
| **6** | Melt Curve 40°C to 95°C, increment 0.5°C for 5 seconds, | | | | |

| **Plate Read** | | | | | |
|---|---|---|---|---|---|
| **7** | 10 | for | 10 | minutes | |
| | **End** | | | | |

### Example 2 Multiplex PCR reaction

The following example shows results of PCR reactions comprising 3 different designs of PTO (tagging probes) specific for the *ipaH* gene, carrying increasing length of MTDR region where DEC500P has the shortest and thereby lowest Tₘ, and DEC503P has the longest MTDR and hence highest Tₘ. All reactions were performed with the 2 common primers (DEC229F and DEC230R). The 3 different tagging probes were tested in combination with a single CQO (quenching probe) design (DEC481rP). The 3 PTO designs were tested alone (figures 9- 14), combining probes DEC500P with DEC502P (fig. 15-16), and combining probes DEC500P and DEC503P (fig 17 -18). The results illustrates that each of the PTO perform well in PCR alone and in combination. In particular, the results furthermore show that combining probes DEC500P with DEC502P provides 2 individually distinguishable melting curves, indicating that both probes detect the specific signal. The results furthermore show, that combining probes DEC500P with DEC503P also provides 2 individually distinguishable melting curves with even wider spaced curves in accordance with the bigger Tm difference between the MTDR of DEC500P and DEC503P while still showing that both probes detect the specific signal. The results further show that all probes provide very low background melting curves in the NTC reaction indicating that the PTO probes do not provide a signal without the presence of the specific target.

**Table 2: Primer and probes sequences (5' to 3'):**

| **PCR** | **Seq ID NO:** | |
|---|---|---|
| **Primers:** | | |
| DEC229F | 1 | ZGTCCATCAGGCATCAGAAGG |
| DEC230R | 2 | ZGGTAGACTTCTATCTCATCCAC |
| **Quenching probe** | | |
| DEC481 rP | 3 | |
| **Tagging Probe** | | |
| DEC500P | 10 | |
| DEC502P | 11 | |
| DEC503P | 12 | |

| | | |
|---|---|---|
| Z= TINA, BHQ1= black hole quencher 1, BHQ2= black hole quencher 2, BHQ3= black hole quencher 3, (dT-FAM)= internal FAM attached to T. (dT-BHQ1)=internal BHQ1 attached to T. (dT-BHQ2)=internal BHQ2 attached to T. phos = phosphate group to block extension. | | |

### PCR reaction:

A 10 ul reaction was prepared that contained 1x GoTaq Probe qPCR mastermix (prod. # Promega A6101), 200 nM forward and reverse primer, 400 nM tagging probe, 800 nM quenching probe, 1:200 dilution of target, 0,25 U Uracil DNA Glycosylase, 1 U/µL, (#EN0361 Fermentas). Target was prepared by mixing a single colony of E. coli carrying the ipaH gene in 200 ul water and boiling 95°C for 15 minutes. 25 ul boiled target was subsequently added to 100 ul sterile water as a target stock solution which was diluted 5x in the final PCR reaction. Reactions were assembled in AB gene SuperPlate 96-well PCR plate (kat.nr. AB2800) and sealed with Optically clear, adhesive, Microseal B film (Bio-Rad, Cat.nr. MSB1001).

PCR reaction mix was subjected to the following PCR cycling and melting curve program (Bio-Rad CFX96 Real-Time PCR Detection System):

### PCR program:

| | **Temp (°C)** | | **Time** | | |
|---|---|---|---|---|---|
| **1** | 40 | For | 10 | minutes | UNG treatment |
| **2** | 95 | For | 10 | minutes | Activation/denaturation |
| **3** | 95 | For | 15 | seconds | a) Denaturation |

| **Plate Read** | | | | | |
|---|---|---|---|---|---|
| **4** | **60** | For | 60 | seconds | b) Annealing/elongation |
| GoTo 3, 39 more times | | | | | |
| **5** | 95 | for | 10 | seconds | |
| **6** | Melt Curve | 40°C to | 95°C, | increment 0.5°C | for 5 seconds, |

| **Plate Read** | | | | | |
|---|---|---|---|---|---|
| **7** | 10 | For | 10 | minutes | |
| | **End** | | | | |

### Example 3 Loop probe design

The following example shows results of PCR reactions comprising 2 different designs of PTO (tagging probes) specific for the ipaH gene, carrying a loop-design as the MTDR region where the last part of the PTO (tagging probe) comprises the targeting portion (quenching probe part), separated from the MTDR region by a loop region. All reactions are performed with the 2 common primers (DEC229F and DEC230R). The 2 different PTOs (tagging probes) were tested alone (RMD7P, figures 19-20 and RMD8P, figures 21-22). The results illustrate that each of the probes performs well in PCR and provide amplification curves in the presence of the specific target only. In addition, the results show that both probes provide a melting curve in the presence of the correct target but no target in the NTC.

**Table 3: Primer and probes sequences (5' to 3'):**

| PCR Primers: | | Seq ID NO: |
|---|---|---|
| DEC229F | ZGTCCATCAGGCATCAGAAGG | 1 |
| DEC230R | ZGGTAGACTTCTATCTCATCCAC | 2 |
| Quenching probe | | |
| DEC481 rP | | 3 |
| Tagging Probe | | |
| RMD7P | | 13 |
| RMD8P | | 14 |

| | | |
|---|---|---|
| Z= TINA, BHQ1= black hole quencher 1, BHQ2= black hole quencher 2, BHQ3= black hole quencher 3, (dT-FAM)= internal FAM attached to T. (dT-BHQ1)=internal BHQ1 attached to T. (dT-BHQ2)=internal BHQ2 attached to T. phos = phosphate group to block extension. | | |

### PCR reaction:

A 10 ul reaction was prepared that contained 1x SsoAdvanced Universal Probes Supermix (prod. #172-5280, Bio-Rad), 200 nM forward and reverse primer, 400 nM tagging probe, 1:200 dilution of target, 0,25 U Uracil DNA Glycosylase, 1 U/µL, (#EN0361 Fermentas). Target was prepared by mixing a single colony of E. coli in 200 ul water and boiling 95°C for 15 minutes. 25 ul boiled target was subsequently added to 100 ul sterile water as a target stock solution which was diluted 5x in the final PCR reaction. Reactions were assembled in AB gene SuperPlate 96-well PCR plate (kat.nr. AB2800) and sealed with Optically clear, adhesive, Microseal B film (Bio-Rad, Cat.nr. MSB1001).

The PCR reaction mix was subjected to the following PCR cycling and melting curve program (Bio-Rad CFX96 Real-Time PCR Detection System):

**Table 4 - PCR program**

| PCR program: | | | | | |
|---|---|---|---|---|---|
| | Temp (°C) | | Time | | |
| 1 | 40 | for | 10 | minutes | UNG treatment |
| 2 | 95 | for | 10 | minutes | Activation/denaturation |
| 3 | 95 | for | 15 | seconds | a) Denaturation |
| | Plate Read | | | | |
| 4 | 60 | for | 60 | seconds | b) Annealing/elongation |

| GoTo 3, 39 more times | | | | | |
|---|---|---|---|---|---|
| 5 | 95 | for | 10 | seconds | |
| 6 | Melt Curve 40°C to 95°C, increment 0.5°C for 5 seconds, | | | | |
| | Plate Read | | | | |
| 7 | 10 | for | 10 | minutes | |
| | End | | | | |

### Example 4: testing a different MTDR

In this experiment we tested some PTO probes having a different sequence than in the previous examples (RMD) and a matching quencher probe (CQO) to elucidate if a different sequence might work less efficiently to bind the polymerase. The primers and the probe target were the same as for the IpaH probes. As was done for the IpaH probe measurements, a normal hydrolysis probe assay was tested, mValidPrime, with RMD PTO probes and quenchers also present in the samples. With this test we want to evaluate if also the qPCR reaction of this assay becomes inhibited due to limited access of polymerase.

### Methods

### qPCR

The experiment was performed on a LightCycler 480 instrument (Roche). gBlocks, used as template for the RMD assay (Table 5) and for the mValidPrime assay, were ordered from IDT. The master mix was TATAA Probe GrandMaster mix. Taq DNA Polymerase was added to the master mix to produce polymerase concentrations 1, 2.5, 5, and 10 times that of normal polymerase concentration in the master mix, where a normal concentration is defined as the concentration indicated by the manufacturer as the optimal concentration for performing the reaction. The qPCR measurement was performed with five probes for the RMD assay (Table 6). Each probe was tested in ten different mixtures, as shown in Table 7. One set of five mixtures, with four different concentrations of polymerase and one NTC, was made with only the RMD system. One set of five other mixtures was made without IpaH primers and template, but with presence of primers, probes and template of the assay ValidPrime for mouse. The reagents were mixed to the concentrations shown in Table 8. All samples were run in duplicates. The temperature program is shown in Table 9. Here the fluorescence was measured both in step 2 (60 °C) and in step 3 (95 °C) of the program. The fluorescence is normally only measured in the 60°C step. Since the probes might be hybridized to the quenching (CQO) probes and the fluorescence might be quenched at 60°C, we here also measured at 95°C where all double stranded DNA is melted.

**Table 5. Sequences of gBlocks.**

| |
|---|
| **RMD sequence (SEQ ID NO: 15):** |
| |

| |
|---|
| Colour code: **Forward primer** Reverse primer *Probe target* |

**Table 6. Sequences of probes and primers**

| *Type* | *Name* | *Length* | *Sequence* | *SEQ ID NO:* |
|---|---|---|---|---|
| forward primer | DEC229F | 20 | GTCCATCAGGCATCAGAAGG | 1 |
| reverse primer | DEC230R | 22 | | 2 |
| quencher probe (CQO) | RMD23rP | 57 | | 16 |
| Tagging probe (PTO) | RMD28P | 46 | | 17 |
| Tagging probe (PTO) | RMD29P | 51 | | 18 |
| Tagging probe (PTO) | RMD30P | 58 | | 19 |
| Tagging probe (PTO) | RMD31P | 74 | | 20 |
| Tagging probe (PTO) | RMD32P | 91 | | 21 |

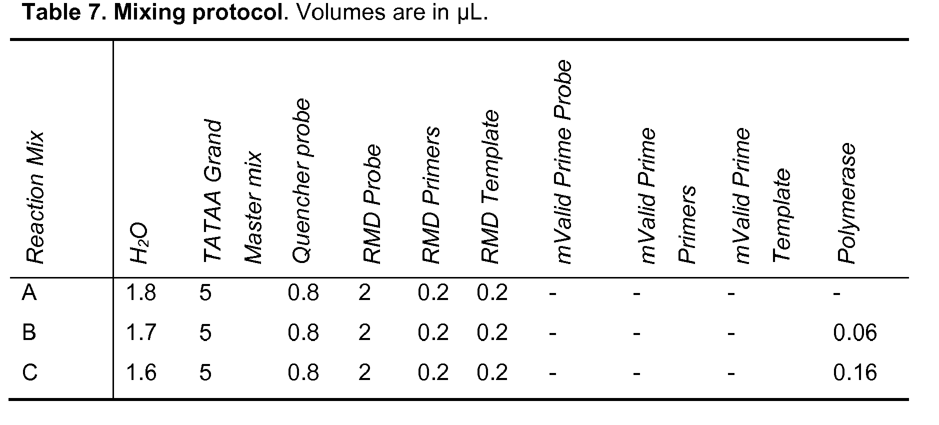

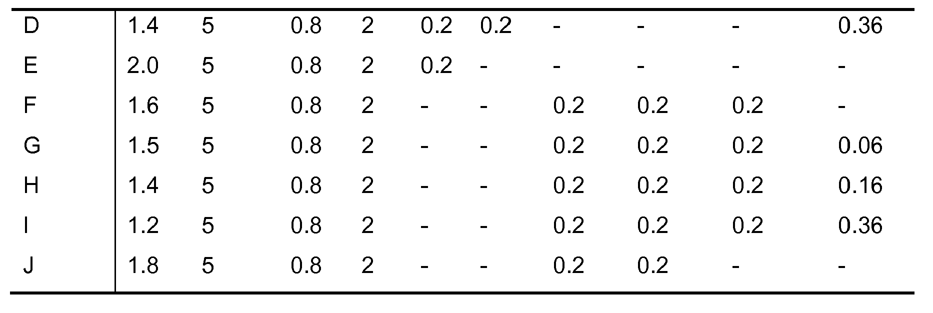

**Table 8. Reagent concentrations**

| *Reagents* | *Working solution conc.* | *Final conc.* |
|---|---|---|
| Primers (forward & reverse) RMD | 10 µM | 200 nM |
| PTO Probe RMD | 2 µM | 400 nM |
| Quencher probe | 10 µM | 800 nM |
| Template (gBlocks) RMD | 1^{∗}10⁶ molecules/µl | 2*10⁴ molecules/µl |
| Primers (forward & reverse) mValidPrime | 10 µM | 200 nM |
| Probe mValidPrime | 10 µM | 200 nM |
| Template (gBlocks) mValidPrime | 1^{∗}10⁶ molecules/µl | 2*10⁴ molecules/µl |
| Master Mix | 2X | 1X |
| Taq polymerase | 5 units / µl | varying |

**Table 9. Temperature program for the probe qPCR run.**

| *Step* | *Time* | *Temperature* | *Cycles* |
|---|---|---|---|
| Activation | 60 s | 95°C | |
| Cycling | 15 s | 95°C (Data acquisition) | 50 |
| Denaturation | | | |
| Extension | 60 s | 60 °C (Data acquisition) | |
| Melt curve | 0.5°C / 10 s | 40°C - 95°C (Data acquisition) | |
| Cooling | 600 s | 10°C | |

### Results

### Cq-values

The replicate averages of the Cq values for the RMD samples (A-D) were calculated. They are plotted as a function of polymerase concentration in Figure 23. Amplification generally increased with increasing concentrations of polymerase.

Figure 24 shows the Cq values of the samples containing the mValidPrime probes. The samples contain also the various RMD probes, but since no primers or templates for these probes were in the samples, only the mValidPrime target is amplified and the fluorescence mainly comes from the mValidPrime probes. Amplification generally increased with increasing concentrations of polymerase.

### Amplification curves

The amplitude of the amplification curves can be adjusted by varying the length of the probes and the polymerase concentration, as shown in Figure 25. This figure shows the fluorescence amplitudes measured in the last amplification cycle in the denaturation step at 95°C. Generally, at higher polymerase concentrations higher amplitudes were reached for all probes.

The amplitude of the mValidPrime probes, Figure 26, were essentially stable over the tested range of polymerase concentrations.

### Melting temperatures

The measured melting temperatures of the RMD probes are found in Figure 27. Melting curves were only observed for the samples containing RMD template, mixtures A, B, C and D. The melting temperatures increased with probe length, and they decreased slightly with increasing polymerase concentration.

### Example 5: target concentration

The following example shows results of PCR reactions comprising 5x dilution curves of 6 different target concentrations detected by the Probing and Tagging oligonucleotide (PTO) DEC486P specific for the *ipaH* gene. Reactions were performed with the 2 common primers (DEC229F and DEC230R). The Probing and Tagging oligonucleotides were tested in combination with a single Capture and Quenching probe design (DEC481rP). The results illustrate that the Probing and Tagging probe works by providing an amplification curve and a melting curve corresponding to the target dilution. As can be seen from the NTC reactions the DEC486P probe showed very little background.

**Table 10: Primer and probes sequences (5' to 3'):**

| **PCR Primers:** | | **Seq ID NO:** |
|---|---|---|
| DEC229F | ZGTCCATCAGGCATCAGAAGG | 1 |
| DEC230R | ZGGTAGACTTCTATCTCATCCAC | 2 |
| **Capturing and Quenching probe** | | |
| DEC481rP | | 3 |
| **Probing and Tagging Probe** | | |
| DEC486P | | 4 |

| | | |
|---|---|---|
| Z= TINA, BHQ1= black hole quencher 1, BHQ2= black hole quencher 2, BHQ3= black hole quencher 3, (dT-FAM)= internal fluorescein label attached to T. FAM=Fluorescein. (dT-BHQ1)=internal BHQ1 attached to T. (dT-BHQ2)=internal BHQ2 attached to T. phos = phosphate group to block extension. | | |

PCR reaction: A 10 µl reaction was prepared that contained 1 x Sso Advanced Universal Probe Supermix (prod. # Promega A6101), 200 nM forward and reverse primer, 400 nM Probing and Tagging probe, 800 nM Capturing and Quenching probe, 1:200 dilution of target, 0,25 U Uracil DNA Glycosylase, 1 U/µL, (#EN0361 Fermentas). Target was prepared by mixing a single colony of E. coli carrying the *ipaH* gene in 200 µl water and boiling 95°C for 15 minutes. 25 µl boiled target was subsequently added to 100 µl sterile water as a target stock solution which was diluted 5 to 15625x in the final PCR reaction. Reactions were assembled in AB gene SuperPlate 96-well PCR plate (kat.nr. AB2800) and sealed with Optically clear, adhesive, Microseal B film (Bio-Rad, Cat.nr. MSB1001).

PCR reaction mix was subjected to the following PCR cycling and melting curve program (Bio-Rad CFX96 Real-Time PCR Detection System):

**Table 11**

| **PCR program:** | | | | | |
|---|---|---|---|---|---|
| | **Temp (°C)** | | **Time** | | |
| **1** | 40 | for | 10 | minutes | UNG treatment |
| **2** | 95 | for | 10 | minutes | Activation/denaturation |
| **3** | 95 | for | 15 | seconds | a) Denaturation |

| **Plate Read** | | | | | |
|---|---|---|---|---|---|
| **4** | **60** | for | 60 | seconds | b) Annealing/elongation |
| GoTo 3, 39 more times | | | | | |
| **5** | 95 | for | 10 | seconds | |
| **6** Melt Curve 40°C to 95°C, increment 0.5°C for 5 seconds, | | | | | |

| **Plate Read** | | | | | |
|---|---|---|---|---|---|
| **7** | 10 | for | 10 | minutes | |
| | **End** | | | | |

### References

Kibbe WA. 'OligoCalc: an online oligonucleotide properties calculator'. (2007) Nucleic Acids Res. 35(webserver issue): May 25.
http://www.basic.northwestern.edu/biotools/oligocalc.html
BioTechniques 38:569-575 (April 2005)
Nucleic Acids Symp Ser (2008) 52(1): 47-48.

## Claims

1. A method for detecting a target nucleic acid sequence, the method comprising the steps of:
(a) hybridizing the target nucleic acid sequence with a PTO (Probing and Tagging Oligonucleotide); the PTO comprising (i) a targeting portion comprising a nucleotide sequence substantially complementary to the target nucleic acid sequence, and (ii) a Melting Temperature Deciding Region (MTDR), comprising a nucleotide sequence non-complementary to the target nucleic acid sequence, and (iii) at least one set of interactive labels comprising at least one fluorophore and at least one quencher;
(b) hybridizing said PTO with a CQO (Capturing and Quenching Oligonucleotide); wherein the CQO comprises (i) a capturing portion comprising a nucleotide sequence which is reverse complementary to the MTDR of the PTO and (ii) at least one quenching molecule; wherein the MTDR of the PTO is configured to hybridize with the capturing portion of the CQO to form a Tag Duplex;
(c) contacting the Tag Duplex with an enzyme having nuclease activity; wherein the enzyme having nuclease activity induces cleavage of the Tag Duplex when the Tag Duplex is hybridized with the target nucleic acid sequence thereby releasing an activated Tag Duplex fragment comprising a PTO fragment comprising the MTDR hybridized to the capturing portion of the CQO and the at least one fluorophore;
(d) melting and/or hybridizing said activated Tag Duplex fragment to obtain a signal from the at least one fluorophore, and
(e) detecting the activated Tag Duplex fragment by measuring the signal from the at least one fluorophore; wherein the signal is indicative of the presence of the target nucleic acid sequence.

2. The method according to claim 1, wherein step (b) is performed prior to step (a) as follows;
(b) hybridizing a PTO with a CQO, wherein the PTO comprises (i) a targeting portion comprising a nucleotide sequence substantially complementary to the target nucleic acid sequence, and (ii) a Melting Temperature Deciding Region (MTDR), comprising a nucleotide sequence non-complementary to the target nucleic acid sequence, and (iii) at least one set of interactive labels comprising at least one fluorophore and at least one quencher; wherein the CQO comprises (i) a capturing portion comprising a nucleotide sequence which is reverse complementary to the MTDR of the PTO and (ii) at least one quenching molecule; wherein the MTDR of the PTO is configured to hybridize with the capturing portion of the CQO to form a Tag Duplex; and
(a) hybridizing the target nucleic acid sequence with said Tag Duplex;
(c) contacting the Tag Duplex with an enzyme having nuclease activity; wherein the enzyme having nuclease activity induces cleavage of the Tag Duplex when the Tag Duplex is hybridized with the target nucleic acid sequence thereby releasing an activated Tag Duplex fragment comprising a PTO fragment comprising the MTDR hybridized to the capturing portion of the CQO and the at least one fluorophore;
(d) melting and/or hybridizing said activated Tag Duplex fragment to obtain a signal from the at least one fluorophore, and
(e) detecting the activated Tag Duplex fragment by measuring the signal from the at least one fluorophore; wherein the signal is indicative of the presence of the target nucleic acid sequence.

3. The method according to claim 1 wherein steps (b) and (c) occur in reverse order a follows:
Step (a) hybridizing a target nucleic acid sequence with a PTO (Probing and Tagging Oligonucleotide); the PTO comprising (i) a targeting portion comprising a nucleotide sequence substantially complementary to the target nucleic acid sequence, and (ii) a Melting Temperature Deciding Region (MTDR), comprising a nucleotide sequence non-complementary to the target nucleic acid sequence, and (iii) at least one set of interactive labels comprising at least one fluorophore and at least one quencher;
Step (c) contacting the hybridized PTO with an enzyme having nuclease activity; wherein the enzyme having nuclease activity induces cleavage of the PTO when the PTO is hybridized with the target nucleic acid sequence thereby releasing an activated PTO fragment comprising the MTDR and the at least one fluorophore;
Step (b) hybridizing said activated PTO with a CQO (Capturing and Quenching Oligonucleotide); wherein the CQO comprises (i) a capturing portion comprising a nucleotide sequence which is reverse complementary to the MTDR of the PTO and (ii) at least one quenching molecule; wherein the MTDR of the PTO is configured to hybridize with the capturing portion of the CQO to form an activated Tag Duplex;
Step (d) melting and/or hybridizing said activated Tag Duplex fragment to obtain a signal from the at least one fluorophore, and
Step (e) detecting the activated Tag Duplex fragment by measuring the signal from the at least one fluorophore; wherein the signal is indicative of the presence of the target nucleic acid sequence.

4. The method according to any one of the preceding claims, wherein the at least one CQO is configured to detect a group of PTOs such as two or more PTOs.

5. The method according to any one of the preceding claims wherein said method is conducted in the presence of an oligonucleotide primer pair, said primer pair comprising a first primer complementary to said target nucleic acid and which primes the synthesis of a first extension product that is complementary to said target nucleic acid, and a second primer complementary to said first extension product and which primes the synthesis of a second extension product.

6. The method according to any of the preceding claims, wherein at least two target nucleic acid sequences can be distinguished from each other based on the difference in melting temperature of their respective activated Tag Duplex fragments.

7. The method according to any one of the preceding claims, wherein the targeting portion and the MTDR of the PTO are separated by a linker molecule, wherein the linker is a nucleic acid linker comprising 1-200 nucleotides, such as 1-50 nucleotides, such as 1-30 nucleotides, such as 2-20 nucleotides, such as 6-13 nucleotides, such as 8-12 nucleotides, such as 9-12 nucleotides, such as 11 nucleotides, and/or wherein the targeting portion and the MTDR of the PTO are separated by a linker molecule, such as a non-nucleic acid linker or a linker molecule comprising nucleic acids and/or non-nucleic acids such as an organic compound.

8. The method according to any one of the preceding claims, wherein the total length of the PTO is between 10 and 500 nucleotides, such as between 20 and 100, such as between 30 and 70 nucleotides and/or the total length of the CQO is between 10 and 500 nucleotides or base pairs, such as between 15 and 100, such as between 20 and 50 nucleotides or base pairs.

9. The method according to any one of the preceding claims, wherein the targeting portion is located in the 5' end of the PTO and/or wherein the MTDR is located in the 3' end of the PTO.

10. The method according to any one of the preceding claims, wherein the PTO and/or CQO further comprises a blocking group in the 3' end, optionally wherein the blocking group is selected from the group consisting of biotin, labels, a phosphate group, alkyl group, non-nucleotide linker, phosphorothioate, and/or alkane-diol and/or wherein the blocking group comprises nucleotide with no 3'-hydroxyl group such as dideoxynucleotide.

11. The method according to any one of the preceding claims, wherein the enzyme having nuclease activity is a template dependent DNA polymerase, optionally a thermostable template dependent DNA polymerase, such as a Taq polymerase.

12. The method, according to any one of the preceding claims, wherein the target nucleic acid sequence is from a pathogenic organism such as a bacterium, virus, fungus, and/or protozoan.

13. A kit of parts for detecting at least one target nucleic acid sequence from a nucleic acid mixture, the kit comprising:
i. at least one PTO, according to any one of the preceding claims, and
ii. at least one CQO, according to any one of the preceding claims, and
iii. optionally instructions on how to detect a target nucleic acid sequence.

14. A reaction mixture for use in a process for the amplification and/or detection of a target nucleic acid sequence in a sample wherein the reaction mixture, prior to amplification, comprises at least one pair of oligonucleotide primers, at least one PTO and at least one CQO, wherein said pair of primers, PTO and CQO are **characterized in that** said pair of oligonucleotide primers comprises a first a primer complementary to said target nucleic acid and which primes the synthesis of a first extension product that is complementary to said target nucleic acid, and a second primer complementary to said first extension product and which primes the synthesis of a second extension product; and said PTO hybridizes to a nucleotide sequence substantially complementary to the target nucleic acid sequence or the complement of said target nucleic acid, wherein said region is between one member of said primer pair and the complement of the other member of said primer pair and the PTO comprises at least one set of interactive labels, a MTDR, and optionally a linker between the targeting portion and the MTDR; and wherein the CQO comprises at least one quencher and a capturing portion, said capturing portion being configured to hybridize to the PTO.

15. The reaction mixture for the use according to claim 14, wherein the detection of a target nucleic acid sequence is performed according to the method of any of claims 1 to 12.

## Patentansprüche

1. Verfahren zum Detektieren einer Zielnukleinsäuresequenz, wobei das Verfahren die folgenden Schritte umfasst:
(a) Hybridisieren der Zielnukleinsäuresequenz mit einem PTO (Probing and Tagging Oligonucleotide); wobei das PTO (i) einen Targeting-Abschnitt, der eine Nukleotidsequenz umfasst, die im Wesentlichen komplementär zu der Zielnukleinsäuresequenz ist, und (ii) eine Schmelztemperaturentscheidungsregion (Melting Temperature Deciding Region - MTDR), die eine Nukleotidsequenz umfasst, die nicht komplementär zu der Zielnukleinsäuresequenz ist, und (iii) mindestens einen Satz interaktiver Markierungen, der mindestens ein Fluorophor und mindestens einen Quencher umfasst, umfasst;
(b) Hybridisieren des PTO mit einem CQO (Capturing and Quenching Oligonukleotide); wobei das CQO (i) einen Einfangabschnitt, der eine Nukleotidsequenz umfasst, die umgekehrt komplementär zu der MTDR des PTO ist, und (ii) mindestens ein Quenchmolekül umfasst; wobei die MTDR des PTO dazu konfiguriert ist, mit dem Einfangabschnitt des CQO zu hybridisieren, um eine Tag-Duplex zu bilden;
(c) Inkontaktbringen der Tag-Duplex mit einem Enzym, das Nukleaseaktivität aufweist; wobei das Enzym, das Nukleaseaktivität aufweist, eine Spaltung der Tag-Duplex induziert, wenn die Tag-Duplex mit der Zielnukleinsäuresequenz hybridisiert ist, wodurch ein aktiviertes Tag-Duplex-Fragment freigesetzt wird, das ein PTO-Fragment umfasst, welches die an den Einfangabschnitt des CQO hybridisierte MTDR und das mindestens eine Fluorophor umfasst;
(d) Schmelzen und/oder Hybridisieren des aktivierten Tag-Duplex-Fragments, um ein Signal von dem mindestens einen Fluorophor zu erhalten, und
(e) Detektieren des aktivierten Tag-Duplex-Fragments durch Messen des Signals von dem mindestens einen Fluorophor; wobei das Signal das Vorhandensein der Zielnukleinsäuresequenz anzeigt.

2. Verfahren nach Anspruch 1, wobei der Schritt (b) vor dem Schritt (a) wie folgt durchgeführt wird;
(b) Hybridisieren eines PTO mit einem CQO, wobei das PTO (i) einen Targeting-Abschnitt, der eine Nukleotidsequenz umfasst, die im Wesentlichen komplementär zu der Zielnukleinsäuresequenz ist, und (ii) eine Schmelztemperaturentscheidungsregion (Melting Temperature Deciding Region - MTDR), die eine Nukleotidsequenz umfasst, die nicht komplementär zu der Zielnukleinsäuresequenz ist, und (iii) mindestens einen Satz interaktiver Markierungen, der mindestens ein Fluorophor und mindestens einen Quencher umfasst, umfasst; wobei das CQO (i) einen Einfangabschnitt, der eine Nukleotidsequenz umfasst, die umgekehrt komplementär zu der MTDR des PTO ist, und (ii) mindestens ein Quenchmolekül umfasst; wobei die MTDR des PTO dazu konfiguriert ist, mit dem Einfangabschnitt des CQO zu hybridisieren, um eine Tag-Duplex zu bilden; und
(a) Hybridisieren der Zielnukleinsäuresequenz mit der Tag-Duplex;
(c) Inkontaktbringen der Tag-Duplex mit einem Enzym, das Nukleaseaktivität aufweist; wobei das Enzym, das Nukleaseaktivität aufweist, eine Spaltung der Tag-Duplex induziert, wenn die Tag-Duplex mit der Zielnukleinsäuresequenz hybridisiert ist, wodurch ein aktiviertes Tag-Duplex-Fragment freigesetzt wird, das ein PTO-Fragment umfasst, welches die an den Einfangabschnitt des CQO hybridisierte MTDR und das mindestens eine Fluorophor umfasst;
(d) Schmelzen und/oder Hybridisieren des aktivierten Tag-Duplex-Fragments, um ein Signal von dem mindestens einen Fluorophor zu erhalten, und
(e) Detektieren des aktivierten Tag-Duplex-Fragments durch Messen des Signals von dem mindestens einen Fluorophor; wobei das Signal das Vorhandensein der Zielnukleinsäuresequenz anzeigt.

3. Verfahren nach Anspruch 1, wobei die Schritte (b) und (c) in umgekehrter Reihenfolge wie folgt erfolgen:
Schritt (a) Hybridisieren einer Zielnukleinsäuresequenz mit einem PTO (Probing and Tagging Oligonucleotide); wobei das PTO (i) einen Targeting-Abschnitt, der eine Nukleotidsequenz umfasst, die im Wesentlichen komplementär zu der Zielnukleinsäuresequenz ist, und (ii) eine Schmelztemperaturentscheidungsregion (Melting Temperature Deciding Region - MTDR), die eine Nukleotidsequenz umfasst, die nicht komplementär zu der Zielnukleinsäuresequenz ist, und (iii) mindestens einen Satz interaktiver Markierungen, der mindestens ein Fluorophor und mindestens einen Quencher umfasst, umfasst;
Schritt (c) Inkontaktbringen des hybridisierten PTO mit einem Enzym, das Nukleaseaktivität aufweist; wobei das Enzym, das Nukleaseaktivität aufweist, eine Spaltung des PTO induziert, wenn das PTO mit der Zielnukleinsäuresequenz hybridisiert ist, wodurch ein aktiviertes PTO-Fragment freigesetzt wird, welches die MTDR und das mindestens eine Fluorophor umfasst;
Schritt (b) Hybridisieren des aktivierten PTO mit einem CQO (Capturing and Quenching Oligonucleotide); wobei das CQO (i) einen Einfangabschnitt, der eine Nukleotidsequenz umfasst, die umgekehrt komplementär zu der MTDR des PTO ist, und (ii) mindestens ein Quenchmolekül umfasst; wobei die MTDR des PTO dazu konfiguriert ist, mit dem Einfangabschnitt des CQO zu hybridisieren, um eine aktivierte Tag-Duplex zu bilden;
Schritt (d) Schmelzen und/oder Hybridisieren des aktivierten Tag-Duplex-Fragments, um ein Signal von dem mindestens einen Fluorophor zu erhalten, und
Schritt (e) Detektieren des aktivierten Tag-Duplex-Fragments durch Messen des Signals von dem mindestens einen Fluorophor;
wobei das Signal das Vorhandensein der Zielnukleinsäuresequenz anzeigt.

4. Verfahren nach einem der vorhergehenden Ansprüche, wobei das mindestens eine CQO dazu konfiguriert ist, eine Gruppe von PTOs nachzuweisen, wie etwa zwei oder mehr PTOs.

5. Verfahren nach einem der vorhergehenden Ansprüche, wobei das Verfahren in Gegenwart eines Oligonukleotid-Primerpaars durchgeführt wird, wobei das Primerpaar einen ersten Primer, der komplementär zu der Zielnukleinsäure ist und der als Primer für die Synthese eines ersten Verlängerungsprodukts dient, das komplementär zu der Zielnukleinsäure ist und einen zweiten Primer umfasst, der komplementär zu dem ersten Verlängerungsprodukt ist und der als Primer für die Synthese eines zweiten Verlängerungsprodukts dient.

6. Verfahren nach einem der vorhergehenden Ansprüche, wobei mindestens zwei Zielnukleinsäuresequenzen basierend auf dem Unterschied der Schmelztemperatur ihrer jeweiligen aktivierten Tag-Duplex-Fragmente voneinander unterschieden werden können.

7. Verfahren nach einem der vorhergehenden Ansprüche, wobei der Targeting-Abschnitt und die MTDR des PTO durch ein Linker-Molekül getrennt sind, wobei der Linker ein Nukleinsäure-Linker ist, der 1-200 Nukleotide, wie etwa 1-50 Nukleotide, wie etwa 1-30 Nukleotide, wie etwa 2-20 Nukleotide, wie etwa 6-13 Nukleotide, wie etwa 8-12 Nukleotide, wie etwa 9-12 Nukleotide, wie etwa 11 Nukleotide umfasst, und/oder wobei der Targeting-Abschnitt und die MTDR des PTO durch ein Linker-Molekül wie etwa einen Nicht-Nukleinsäure-Linker oder ein Linker-Molekül, das Nukleinsäuren und/oder Nicht-Nukleinsäuren wie etwa eine organische Verbindung umfasst, getrennt sind.

8. Verfahren nach einem der vorhergehenden Ansprüche, wobei die Gesamtlänge des PTO zwischen 10 und 500 Nukleotide, wie etwa zwischen 20 und 100, wie etwa zwischen 30 und 70 Nukleotide beträgt und/oder die Gesamtlänge des CQO zwischen 10 und 500 Nukleotide oder Basenpaare, wie etwa zwischen 15 und 100, wie etwa zwischen 20 und 50 Nukleotide oder Basenpaare beträgt.

9. Verfahren nach einem der vorhergehenden Ansprüche, wobei sich der Targeting-Abschnitt an dem 5'-Ende des PTO befindet und/oder sich die MTDR an dem 3'-Ende des PTO befindet.

10. Verfahren nach einem der vorhergehenden Ansprüche, wobei das PTO und/oder das CQO ferner eine Blockierungsgruppe an dem 3'-Ende umfassen/umfasst, wahlweise wobei die Blockierungsgruppe aus der Gruppe bestehend aus Biotin, Markierungen, einer Phosphatgruppe, einer Alkylgruppe, einem Nicht-Nukleotid-Linker, einem Phosphorothioat und/oder einem Alkandiol ausgewählt ist und/oder wobei die Blockierungsgruppe ein Nukleotid ohne 3'-Hydroxylgruppe wie etwa Didesoxynukleotid umfasst.

11. Verfahren nach einem der vorhergehenden Ansprüche, wobei das Enzym, das Nukleaseaktivität aufweist, eine templatabhängige DNA-Polymerase ist, wahlweise eine thermostabile, templatabhängige DNA-Polymerase, wie etwa eine Taq-Polymerase.

12. Verfahren nach einem der vorhergehenden Ansprüche, wobei die Zielnukleinsäuresequenz von einem pathogenen Organismus wie etwa einem Bakterium, Virus, Pilz und/oder Protozoon stammt.

13. Kit aus Teilen zum Detektieren mindestens einer Zielnukleinsäuresequenz aus einem Nukleinsäuregemisch, wobei das Kit Folgendes umfasst:
i. mindestens ein PTO nach einem der vorhergehenden Ansprüche, und
ii. mindestens ein CQO nach einem der vorhergehenden Ansprüche, und
iii. wahlweise Anweisungen, wie eine Zielnukleinsäuresequenz nachgewiesen wird.

14. Reaktionsgemisch zur Verwendung in einem Prozess zur Amplifikation und/oder zur Detektion einer Zielnukleinsäuresequenz in einer Probe, wobei das Reaktionsgemisch vor der Amplifikation mindestens ein Oligonukleotid-Primerpaar, mindestens ein PTO und mindestens ein CQO umfasst, wobei das Primerpaar, das PTO und das CQO **dadurch gekennzeichnet sind, dass** das Oligonukleotid-Primerpaar einen ersten Primer, der komplementär zu der Zielnukleinsäure ist und der als Primer für die Synthese eines ersten Verlängerungsprodukts dient, das komplementär zu der Zielnukleinsäure ist und einen zweiten Primer umfasst, der komplementär zu dem ersten Verlängerungsprodukt ist und der als Primer für die Synthese eines zweiten Verlängerungsprodukts dient; und das PTO mit einer Nukleotidsequenz hybridisiert, die im Wesentlichen komplementär zu der Zielnukleinsäuresequenz oder dem Komplement der Zielnukleinsäure ist, wobei die Region zwischen einem Element des Primerpaars und dem Komplement des anderen Elements des Primerpaars liegt und das PTO mindestens einen Satz interaktiver Markierungen, eine MTDR und wahlweise einen Linker zwischen dem Targeting-Abschnitt und der MTDR umfasst; und wobei das CQO mindestens einen Quencher und einen Einfangabschnitt umfasst, wobei der Einfangabschnitt dazu konfiguriert ist, mit dem PTO zu hybridisieren.

15. Reaktionsgemisch zur Verwendung nach Anspruch 14, wobei die Detektion einer Zielnukleinsäuresequenz nach dem Verfahren nach einem der Ansprüche 1 bis 12 durchgeführt wird.

## Revendications

1. Procédé de détection d'une séquence d'acide nucléique cible, le procédé comprenant les étapes :
(a) d'hybridation de la séquence d'acide nucléique cible avec un PTO (oligonucléotide de sondage et d'étiquetage) ; le PTO comprenant (i) une partie de ciblage comprenant une séquence nucléotidique sensiblement complémentaire à la séquence d'acide nucléique cible, et (ii) une région de décision de la température de fusion (MTDR), comprenant une séquence nucléotidique non complémentaire à la séquence d'acide nucléique cible, et (iii) au moins un ensemble d'étiquettes interactives comprenant au moins un fluorophore et au moins un extincteur ;
(b) d'hybridation dudit PTO avec un CQO (oligonucléotide de capture et d'extinction) ; dans lequel le CQO comprend (i) une partie de capture comprenant une séquence nucléotidique qui est inversement complémentaire au MTDR du PTO et (ii) au moins une molécule d'extinction ; dans lequel le MTDR du PTO est configuré pour s'hybrider avec la partie de capture du CQO pour former un duplex d'étiquette ;
(c) de mise en contact du duplex d'étiquette avec une enzyme ayant une activité nucléase ; dans lequel l'enzyme ayant une activité nucléase induit le clivage du duplex d'étiquette lorsque le duplex d'étiquette est hybridé avec la séquence d'acide nucléique cible, libérant ainsi un fragment de duplex d'étiquette activé comprenant un fragment de PTO comprenant le MTDR hybridé à la partie de capture du CQO et l'au moins un fluorophore ;
(d) de fusion et/ou d'hybridation dudit fragment de duplex d'étiquette activé pour obtenir un signal provenant de l'au moins un fluorophore, et
(e) de détection du fragment de duplex d'étiquette activé par la mesure du signal provenant de l'au moins un fluorophore ; dans lequel le signal indique la présence de la séquence d'acide nucléique cible.

2. Procédé selon la revendication 1, dans lequel l'étape (b) est effectuée avant l'étape (a) comme suit ;
(b) l'hybridation d'un PTO avec un CQO, dans lequel le PTO comprend (i) une partie de ciblage comprenant une séquence nucléotidique sensiblement complémentaire à la séquence d'acide nucléique cible, et (ii) une région de décision de la température de fusion (MTDR), comprenant une séquence nucléotidique non complémentaire à la séquence d'acide nucléique cible, et (iii) au moins un ensemble de marqueurs interactifs comprenant au moins un fluorophore et au moins un extincteur ; dans lequel le CQO comprend (i) une partie de capture comprenant une séquence nucléotidique qui est inversement complémentaire au MTDR du PTO et (ii) au moins une molécule d'extinction ; dans lequel le MTDR du PTO est configuré pour s'hybrider avec la partie de capture du CQO pour former un duplex d'étiquette ; et
(a) l'hybridation de la séquence d'acide nucléique cible avec ledit duplex d'étiquette ;
(c) la mise en contact du duplex d'étiquette avec une enzyme ayant une activité nucléase ; dans lequel l'enzyme ayant une activité nucléase induit le clivage du duplex d'étiquette lorsque le duplex d'étiquette est hybridé avec la séquence d'acide nucléique cible, libérant ainsi un fragment de duplex d'étiquette activé comprenant un fragment de PTO comprenant le MTDR hybridé à la partie de capture du CQO et l'au moins un fluorophore ;
(d) la fusion et/ou l'hybridation dudit fragment de duplex d'étiquette activé pour obtenir un signal provenant de l'au moins un fluorophore, et
(e) la détection du fragment de duplex d'étiquette activé par la mesure du signal provenant de l'au moins un fluorophore ; dans lequel le signal indique la présence de la séquence d'acide nucléique cible.

3. Procédé selon la revendication 1, dans lequel les étapes (b) et (c) se déroulent dans l'ordre inverse comme suit :
Étape (a) d'hybridation d'une séquence d'acide nucléique cible avec un PTO (oligonucléotide de sondage et d'étiquetage) ; le PTO comprenant (i) une partie de ciblage comprenant une séquence nucléotidique sensiblement complémentaire à la séquence d'acide nucléique cible, et (ii) une région de décision de la température de fusion (MTDR), comprenant une séquence nucléotidique non complémentaire à la séquence d'acide nucléique cible, et (iii) au moins un ensemble d'étiquettes interactives comprenant au moins un fluorophore et au moins un extincteur ;
Étape (c) de mise en contact du PTO hybridé avec une enzyme ayant une activité nucléase ; dans lequel l'enzyme ayant une activité nucléase induit le clivage du PTO lorsque le PTO est hybridé avec la séquence d'acide nucléique cible libérant ainsi un fragment de PTO activé comprenant le MTDR et l'au moins un fluorophore ;
Étape (b) d'hybridation dudit PTO activé avec un CQO (oligonucléotide de capture et d'extinction) ; dans lequel le CQO comprend (i) une partie de capture comprenant une séquence nucléotidique qui est inversement complémentaire au MTDR du PTO et (ii) au moins une molécule d'extinction ; dans lequel le MTDR du PTO est configuré pour s'hybrider avec la partie de capture du CQO pour former un duplex d'étiquette activé ;
Étape (d) de fusion et/ou d'hybridation dudit fragment de duplex d'étiquette activé pour obtenir un signal provenant de l'au moins un fluorophore, et
Étape (e) de détection du fragment de duplex d'étiquette activé par la mesure du signal provenant de l'au moins un fluorophore ;
dans lequel le signal indique la présence de la séquence d'acide nucléique cible.

4. Procédé selon l'une quelconque des revendications précédentes, dans lequel l'au moins un CQO est configuré pour détecter un groupe de PTO tel que deux PTO ou plus.

5. Procédé selon l'une quelconque des revendications précédentes, dans lequel ledit procédé est conduit en présence d'une paire d'amorces oligonucléotidiques, ladite paire d'amorces comprenant une première amorce complémentaire audit acide nucléique cible et qui amorce la synthèse d'un premier produit d'extension qui est complémentaire audit acide nucléique cible, et une seconde amorce complémentaire audit premier produit d'extension et qui amorce la synthèse d'un second produit d'extension.

6. Procédé selon l'une quelconque des revendications précédentes, dans lequel au moins deux séquences d'acide nucléique cibles peuvent être distinguées l'une de l'autre sur la base de la différence de température de fusion de leurs fragments de duplex d'étiquette activés respectifs.

7. Procédé selon l'une quelconque des revendications précédentes, dans lequel la partie de ciblage et le MTDR du PTO sont séparés par une molécule de liaison, dans lequel le lieur est un lieur d'acide nucléique comprenant 1 à 200 nucléotides, tel que 1 à 50 nucléotides, tel que 1 à 30 nucléotides, tel que 2 à 20 nucléotides, tel que 6 à 13 nucléotides, tel que 8 à 12 nucléotides, tel que 9 à 12 nucléotides, tel que 11 nucléotides, et/ou dans lequel la partie de ciblage et le MTDR du PTO sont séparés par une molécule de liaison, telle qu'une lieur d'acide non nucléique ou une molécule de liaison comprenant des acides nucléiques et/ou des acides non nucléiques tels qu'un composé organique.

8. Procédé selon l'une quelconque des revendications précédentes, dans lequel la longueur totale du PTO est comprise entre 10 et 500 nucléotides, tel qu'entre 20 et 100, tel qu'entre 30 et 70 nucléotides et/ou la longueur totale du CQO est comprise entre 10 et 500 nucléotides ou paires de bases, tel qu'entre 15 et 100, tels qu'entre 20 et 50 nucléotides ou paires de bases.

9. Procédé selon l'une quelconque des revendications précédentes, dans lequel la partie de ciblage est située à l'extrémité 5' du PTO et/ou dans lequel le MTDR est situé à l'extrémité 3' du PTO.

10. Procédé selon l'une quelconque des revendications précédentes, dans lequel le PTO et/ou le CQO comprend en outre un groupe de blocage à l'extrémité 3', éventuellement dans lequel le groupe de blocage est choisi dans le groupe constitué de biotine, de marqueurs, d'un groupe phosphate, d'un groupe alkyle, d'un lieur non nucléotidique, du phosphorothioate et/ou de l'alcane-diol et/ou dans lequel le groupe de blocage comprend un nucléotide sans groupe 3'-hydroxyle tel qu'un didésoxynucléotide.

11. Procédé selon l'une quelconque des revendications précédentes, dans lequel l'enzyme ayant une activité nucléase est une ADN polymérase dépendant de la matrice, éventuellement une ADN polymérase dépendant de la matrice thermostable, par exemple une polymérase de type Taq.

12. Procédé selon l'une quelconque des revendications précédentes, dans lequel la séquence d'acide nucléique cible provient d'un organisme pathogène tel qu'une bactérie, un virus, un champignon et/ou un protozoaire.

13. Kit de pièces pour détecter au moins une séquence d'acide nucléique cible à partir d'un mélange d'acide nucléique, le kit comprenant :
i. au moins un PTO, selon l'une quelconque des revendications précédentes, et
ii. au moins un CQO, selon l'une quelconque des revendications précédentes, et
iii. éventuellement des instructions sur la façon de détecter une séquence d'acide nucléique cible.

14. Mélange réactionnel destiné à être utilisé dans un procédé d'amplification et/ou de détection d'une séquence d'acide nucléique cible dans un échantillon, dans lequel le mélange réactionnel, avant l'amplification, comprend au moins une paire d'amorces oligonucléotidiques, au moins un PTO et au moins un CQO, dans lequel ladite paire d'amorces, ledit PTO et ledit CQO sont **caractérisés en ce que** ladite paire d'amorces oligonucléotidiques comprend une première amorce complémentaire audit acide nucléique cible et qui amorce la synthèse d'un premier produit d'extension qui est complémentaire audit acide nucléique cible, et une seconde amorce complémentaire audit premier produit d'extension et qui amorce la synthèse d'un second produit d'extension ; et ledit PTO s'hybride avec une séquence nucléotidique sensiblement complémentaire à la séquence d'acide nucléique cible ou au complément dudit acide nucléique cible, dans lequel ladite région est entre un élément de ladite paire d'amorces et le complément de l'autre élément de ladite paire d'amorces et le PTO comprend au moins un ensemble d'étiquettes interactives, un MTDR et éventuellement un lieur entre la partie de ciblage et le MTDR ; et dans lequel le CQO comprend au moins un extincteur et une partie de capture, ladite partie de capture étant configurée pour s'hybrider avec le PTO.

15. Mélange réactionnel destiné à être utilisé selon la revendication 14, dans lequel la détection d'une séquence d'acide nucléique cible est effectuée selon le procédé de l'une quelconque des revendications 1 à 12.
